# EUROPEAN PATENT APPLICATION

(11) **EP 1 623 968 A1**
(43) Date of publication of application: **08.02.2006**
(21) Application number: 04730688.1
(22) Date of filing: 30.04.2004
(51) Int. Cl.: C07C 13/72, C09K 11/06, H05B 33/14

(54) **COMPOUND HAVING SPIRO BOND, MATERIAL FOR LUMINESCENT COATING FORMATION AND ORGANIC ELECTROLUMINESCENCE ELEMENT INCLUDING THE SAME**

(30) Priority: 15.05.2003 JP 2003136838
(71) Applicant: IDEMITSU KOSAN CO., LTD., Tokyo 100-8321 (JP)
(72) Inventor: INOUE, Tetsuya, 2990205 (JP); IKEDA, Hidetsugu, 2990205 (JP); HOSOKAWA, Chishio, 2990205 (JP)
(74) Representative: Gille Hrabal Struck Neidlein Prop Roos
(86) International application number: PCT/JP2004/006331
(87) International publication number: WO 2004/110968

(57) **Abstract**

A novel compound having a spiro bond with a specific structure, a material for forming a luminous coated film comprising the compound having the spiro bond with the specific structure, and an organic electroluminescence device which comprises at least one organic thin film layer sandwiched between a pair of electrode consisting of an anode and a cathode, wherein the organic thin film layer comprises the compound having the spiro bond. The organic EL device employing either the compound having a spiro bond or the material for forming luminous coated film in accordance with the present invention is superior in heat resistance, has stability of the thin film composing the device, emits uniform blue light, and exhibits an excellent luminance, current efficiency even under a low driving voltage.

## Description

### TECHNICAL FIELD

The present invention relates to a compound having spiro bond, a material for luminescent coating formation and an organic electroluminescence element including the same. Particularly, the present invention relates to an organic electroluminescence device excellent in heat resistance, having a thin film with great stability, emitting uniform blue light, exhibiting a great luminance of emitted light and a great efficiency of emitted light under low driving voltage. The present invention also relates to a compound having a spiro bond and a material for forming a luminous coated film both for realizing the organic electroluminescence device.

### BACKGROUND ART

An organic electroluminescence ("electroluminescence" will be occasionally referred to as "EL", hereinafter) device is a spontaneous light emitting device which utilizes the principle that a fluorescent substance emits light by energy of recombination of holes injected from an anode and electrons injected from a cathode when an electric field is applied. Since an organic EL device of the laminate type driven under a low electric voltage was reported by C. W. Tang et al. of Eastman Kodak Company (C. W. Tang and S. A. Vanslyke, Applied Physics Letters, Volume 51, Pages 913, 1987), many studies have been conducted on organic EL devices using organic materials as the constituting materials. Tang et al. used a laminate structure using tris(8-hydroxyquinolinol aluminum) for the light emitting layer and a triphenyldiamine derivative for the hole transporting layer. Advantages of the laminate structure are that the efficiency of hole injection into the light emitting layer can be increased, that the efficiency of forming excited particles which are formed by blocking and recombining electrons injected from the cathode can be increased, and that excited particles formed among the light emitting layer can be enclosed. As the structure of the organic EL device, a two-layered structure having a hole transporting (injecting) layer and an electron transporting and light emitting layer and a three-layered structure having a hole transporting (injecting) layer, a light emitting layer and an electron transporting (injecting) layer are well known. To increase the efficiency of recombination of injected holes and electrons in the devices of the laminate type, the structure of the device and the process for forming the device have been studied.

As the light emitting material of the organic EL device, chelate complexes such as tris(8-quinolinolato)aluminum, coumarine derivatives, tetraphenylbutadiene derivatives, bisstyrylarylene derivatives and oxadiazole derivatives are known. It is reported that light in the visible region ranging from blue light to red light can be obtained by using these light emitting materials, and development of a device exhibiting color images is expected (refer to, for example, Japanese Patent Application Laid-Open Nos. Heisei 8(1996)-239655, Heisei 7(1995)-138561 and Heisei 3(1991)-200289). A device using a phenylanthracene derivative as the light emitting material is disclosed in Japanese Patent Application Laid-Open No. Heisei 8(1996)-012600. Although the anthracene derivative is used as the material for emitting blue light, a further improvement in the efficiency of light emission has been desired.

On the other hand, an improvement in the stability of the thin film has been desired so that the lifetime of the device is increased. However, conventional compounds for the material for emitting blue light tend to form crystals in many cases to cause fracture of the thin film, and the improvement has been desired. For example, a dinaphthylanthracene compound is disclosed in the United States Patent No. 0593571. However, since this compound has a symmetric molecular structure in the horizontal and vertical directions, the molecules are easily arranged to form crystals during storage at high temperatures and driving at high temperatures. Japanese Patent Application Laid-Open No. 2000-273056 discloses an allylanthracene compound asymmetric in the horizontal direction. However, one of the groups as substituents to the anthracendiyl group is a simple group such as phenyl group and biphenyl group, and the crystallization cannot be prevented. Accordingly, the crystallization is regulated by introducing a spiro structure into polymers or molecular structure of low molecular weight, resultantly improving stability of thin-film. (refer to, for example, Polymer Preprints 38 (1997) 349; Japanese PCT publication Nos. 2000-508686 and 2000-504774; Japanese Unexamined Patent Application Laid-Open No. 2002-121547; and International PCT publication No. WO 03/08475.)

However, the polymer-based material in those compounds reveals low light emitting property because of an existence of impurities or unreacted moieties at the polymer end. Further, with regard to the low molecular weight-based material, there are many problems such as high driving voltage about the EL device or an exhibition of color tone variation caused by thermal decomposed substance contamination during vapor deposition film formation. As thus described, there is few blue light emitting material capable of providing a stable EL device with high reliability in the present circumstances.

### DISCLOSURE OF THE INVENTION

The present invention has been made to overcome the above problems and has an object of providing an organic electroluminescence device excellent in heat resistance, having a thin film with great stability, emitting uniform blue light, exhibiting a great luminance of emitted light and a great efficiency of emitted light under low driving voltage. The present invention also has an object of providing both a novel compound having a spiro bond and a material for forming a luminous coated film for realizing the organic electroluminescence device.

As a result of intensive researches and studies to achieve the above object by the present inventors, it was found that employing a novel compound containing a spiro bond represented by following general formula (1) as an light emitting material enables to provide an organic electroluminescence device excellent in heat resistance, having a thin film with great stability, emitting uniform blue light, exhibiting a great luminance of emitted light and a great efficiency of emitted light at low driving voltage. Further, it was found that the compound having a spiro bond exhibits excellent solubility for organic solvents revealing applicability for wet film-forming process such as a spin coating process. Such being the case, the present invention has been accomplished on the basis of the foregoing findings and information.

Namely, the present invention provides a compound having a spiro bond represented by a following general formula (1):

(Sp―)ₙ X(―Y)ₘ (1)

In the general formula (1), Sp is a group having a spiro bond represented by a following general formula (2):

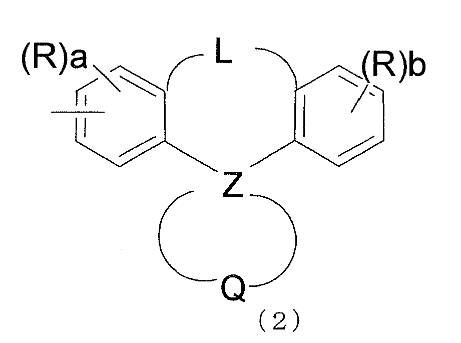

In the general formula (2), L represents a single bond, ―(CR'R")ₑ ―, ― (Si R'R") ₑ ―, ―O―, ―CO― or ―NR'―.
wherein R' and R" each independently represents a hydrogen atom, a substituted or unsubstituted aromatic group having 6 to 50 ring carbon atoms, a substituted or unsubstituted heterocyclic group having 5 to 50 ring atoms, or a substituted or unsubstituted alkyl group having 1 to 50 carbon atoms; e represents an integer of 1 to 10; further R' and R" may be the same with or different from each other;
Z represents a carbon atom, a silicon atom or a germanium atom;
Q represents a group forming a ring structure;
R represents a substituted or unsubstituted aromatic group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, a carboxyl group, a halogen atom, a cyano group, a nitro group or a hydroxyl group; when there are plural of R, they may be the same with or different from each other and they may be bonded with each other to form a ring structure;
a and b each independently represents an integer of 0 to 4 respectively; and
X represents a substituted or unsubstituted aromatic group having 6 to 50 ring carbon atoms, a substituted or unsubstituted condensed aromatic ring group having 12 to 20 ring carbon atoms, or a substituted or unsubstituted aromatic heterocyclic group having 5 to 50 ring carbon atoms or may be a group formed by combining plural of the preceding groups. However, X is not an anthracendiyl group or a polyanthracendiyl group.

In the general formula (1), Y represents a substituted or unsubstituted aromatic group having 6 to 50 ring carbon atoms and may further having a vinyl-bond and still further may contain a group having a spiro bond represented by the general formula (2);
n represents an integer of 1 to 4; and
m represents an integer of 0 to 2.

However, when Sp in the general formula (1) is a spirobifluorenyl group, a case where X has a backbone structure selected from a group consisting of pyrenylene backbone structure, chrysenylene backbone structure and phenanthlene backbone structure is excluded.

Moreover, the present invention provides a material for forming a luminous coated film comprising the above compound containing a spiro bond; and provides an organic EL device comprising at least one of organic thin film layers including a light emitting layer sandwiched between a pair of electrode consisting of an anode and a cathode, wherein the organic EL device comprises the foregoing compound containing a spiro bond of the invention in at least one of the organic compound layers.

### THE PREFERRED EMBODIMENT TO CARRY OUT THE INVENTION

The present invention provides a compound containing a spiro bond represented by a following general formula (1):

(Sₚ―)ₙ X(―Y)ₘ (1)

In the general formula (1), Sp is a group having a spiro bond represented by a following general formula (2):

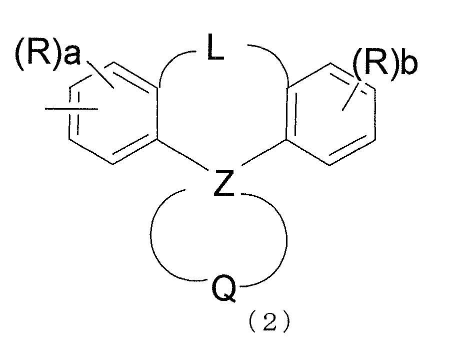

In the general formula (2), Z represents a carbon atom, a silicon atom or a germanium atom.

In the general formula (2), Q represents a group forming a ring structure.

In the general formula (2), R represents a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, a carboxyl group, a halogen atom, a cyano group, a nitro group or a hydroxyl group. When there are plural of R, they may be the same with or different from each other and they may be bonded with each other to form a ring structure.

Examples of the substituted or unsubstituted aromatic group having 6 to 50 ring carbon atoms represented by R include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl) phenyl group, 3-methyl-2-naphthyl group, 4-methyl-1-naphthyl group, 4-methyl-1-anthryl group, 4'-methylbiphenyl-yl group, 4"-t-butyl-p-terphenyl-4-yl group, etc.

Examples of the substituted or unsubstituted aromatic heterocyclic group having 5 to 50 ring atoms represented by R include 1-pyrrolyl group, 2-pyrrolyl group, 3-pyrrolyl group, pyradinyl group, pyrimidyl group, pyridazyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxanyl group, 5-quinoxanyl group, 6-quinoxanyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 10-phenothiazinyl group, 1-phenoxazinyl group, 2-phenoxazinyl group, 3-phenoxazinyl group, 4-phenoxazinyl group, 10-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methyl-pyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group and 4-t-butyl-3-indolyl group, etc.

Examples of the substituted or unsubstituted alkyl group having 1 to 50 carbon atoms represented by R include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxy-isopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyano-propyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, cyclopropyl group, cyclobutyl group, cyclopentyl group, cyclohexyl group, 4-methylcyclohexyl group, 1-adamanthyl group, 2-adamanthyl group, 1-norbornyl group, 2-norbornyl group, etc.

The substituted or unsubstituted alkoxyl group having 1 to 50 carbon atoms represented by R is a group represented by - OY₁. Examples of the group represented by Y₁ include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxy-isopropyl group, 2,3-dihydroxy- t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, etc.

Examples of the substituted or unsubstituted aralkyl group having 7 to 50 carbon atoms represented by R include benzyl group, 1-phenylethyl group, 2-phenylethyl group, 1-phenyl-isopropyl group, 2-phenylisopropyl group, phenyl-t-butyl group, α -naphthylmethyl group, 1- α -naphthylethyl group, 2- α -naphthylethyl group, 1- α -naphthylisopropyl group, 2- α -naphthylisopropyl group, β -naphthylmethyl group, 1- β -naphthylethyl group, 2- β -naphthylethyl group, 1- β -naphthylisopropyl group, 2- β -naphthylisopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group, 1-chloro-2-phenyhsopropyl group, etc.

The substituted or unsubstituted aryloxyl group having 5 to 50 ring carbon atoms represented by R is a group represented by - OY₂. Examples of the group represented by Y₂ include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4"-t-butyl-p-terphenyl-4-yl group, 2-pyrrolyl group, 3-pyrrolyl group, pyradinyl group, 2-pyridinyl group, 3-pyridinyl group, 4-pyridinyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxanyl group, 5-quinoxanyl group, 6-quinoxanyl group, 1-carbazolyl group, 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 1-phenoxazinyl group, 2-phenoxazinyl group, 3-phenoxazinyl group, 4-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methyl- pyrrol-5-yl group, 3-methylpyrrol-1-yl group, 3-methyl-pyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, 4-t-butyl-3-indolyl group, etc.

The substituted or unsubstituted arylthio group having 5 to 50 carbon atoms represented by R is a group represented by - SY₃. Examples of the group represented by Y₃ include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4"-t-butyl-p-terphenyl-4-yl group, 2-pyrrolyl group, 3-pyrrolyl group, pyradinyl group, 2-pyridinyl group, 3-pyridinyl group, 4-pyridinyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxanyl group, 5-quinoxanyl group, 6-quinoxanyl group, 1-carbazolyl group, 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 1-phenoxazinyl group, 2-phenoxazinyl group, 3-phenoxazinyl group, 4-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methyl- pyrrol-5-yl group, 3-methylpyrrol-1-yl group, 3-methyl-pyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, 4-t-butyl-3-indolyl group, etc.

The substituted or unsubstituted alkoxycarbonyl group is represented by ―COOZ₁. Examples of Z₁ include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxy isobutyl group, 1,2-dihydroxy ethyl group, 1,3-dihydroxy isopropyl group, 2,3-dihydroxy-t-butyl group, 1,2,3-trihydroxy propyl group, chloromethyl group, 1-chloroethyl group, 2-chloroethyl group, 2-chloroisobutyl group, 1,2-dichloroethyl group, 1,3-dichloro isopropyl group, 2,3-dichloro-t-butyl group, 1,2,3-trichloro propyl group, bromomethyl group, 1-bromoethyl group, 2-bromoethyl group, 2-bromo isobutyl group, 1,2-dibromo ethyl group, 1,3-dibromo isopropyl group, 2,3-dibromo-t-butyl group, 1,2,3-tribromo propyl group, iodo methyl group, 1-iodo ethyl group, 2-iodo ethyl group, 2-iodo isobutyl group, 1,2-diiodo ethyl group, 1,3-diiodo isopropyl group, 2,3-diiodo-t-butyl group, 1,2,3-triiodo propyl group, aminomethyl group, 1-amino ethyl group, 2-amino ethyl group, 2-amino isobutyl group, 1,2-diamino ethyl group, 1,3-diamino isopropyl group, 2,3-diamino-t-butyl group, 1,2,3-triamino propyl group, cyanomethyl group, 1-cyanoethyl group, 2-cyanoethyl group, 2-cyano isobutyl group, 1,2-dicyano ethyl group, 1,3-dicyano isopropyl group, 2,3-dicyano -t-butyl group, 1,2,3-tricyano propyl group, nitromethyl group, 1-nitroethyl group, 2-nitroethyl group, 2-nitro isobutyl group, 1,2-dinitro ethyl group, 1,3-dinitro isopropyl group, 2,3-dinitro-t-butyl group, 1,2,3-trinitro propyl group, etc.

In the general formula (2), a and b each independently represents an integer of 0 to 4, and preferably an integer of 0 to 2 respectively.

In the general formula (2), L represents a single bond, ―(CR'R")ₑ―, ―(Si R'R")ₑ―, ―O―, ―CO― or -NR'-;
wherein R' and R" each independently represents a hydrogen atom, a substituted or unsubstituted aromatic group having 6 to 50 ring carbon atoms, a substituted or unsubstituted heterocyclic group having 5 to 50 ring atoms, or a substituted or unsubstituted alkyl group having 1 to 50 carbon atoms; specific examples of those groups are the same as explained about the above R in the foregoing description; and
e represents an integer of 1 to 10; further R' and R" may be the same with or different from each other.

In the general formula (1), X represents a substituted or unsubstituted aromatic group having 6 to 50 ring carbon atoms, a substituted or unsubstituted condensed aromatic ring group having 12 to 20 ring carbon atoms, or a substituted or unsubstituted aromatic heterocyclic group having 5 to 50 ring atoms or may be a group formed by combining plural of the preceding groups. However, X is not an anthracene diyl group or a polyanthracene diyl group.

Specific examples of the substituted or unsubstituted aromatic group having 6 to 50 ring carbon atoms and the substituted or unsubstituted aromatic heterocyclic group having 5 to 50 ring atoms both represented by X are the same as those described about the aforementioned R.

Examples of the substituted or unsubstituted condensed aromatic ring group having 12 to 20 ring carbon atoms represented by X include phenanthrene group, fluoranthene group, pyrene group, perylene group, coronene group, chrysene group, picene group, fluorene group, terphenyl group, biphenyl group, N-alkyl group or a bivalent form of aryl carbazole group, triphenylene group, rubicene group, etc.

In the general formula (1), Y represents a substituted or unsubstituted aromatic group having 6 to 50 ring carbon atoms and may further having a vinyl-bond and still further may contain a group having a spiro bond represented by the general formula (2).

Examples of the substituted or unsubstituted aromatic group having 6 to 50 ring carbon atoms and may further having a vinyl-bond represented by Y include the same as those described about the aforementioned R and those made by combining with a substituted or unsubstituted vinyl group. Specific examples are shown as follows:

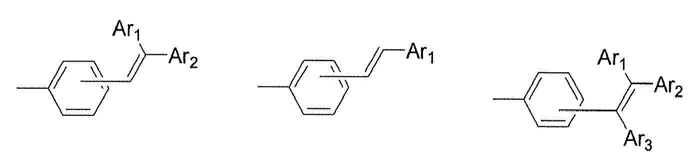

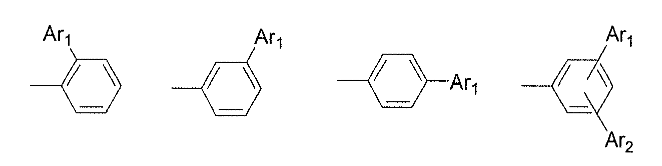

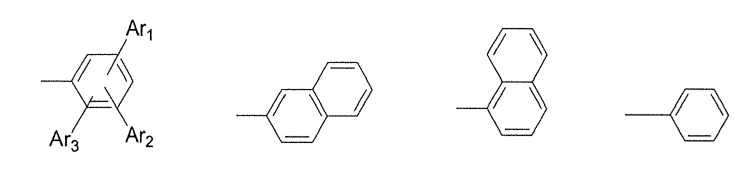

In the above groups, Ar₁ to Ar₃ each independently represents a substituted or unsubstituted aromatic group having 6 to 50 ring carbon atoms respectively and specific examples are the same as those described about the aforementioned R. Further, Ar₁ to Ar₃ may be the same with or different from each other.

Among those, it is preferable that Y is a group represented by a following general formula (26):

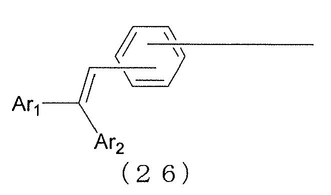

Furthermore, Y may be selected from the groups represented by the general formula (2) (preferably general formulae (4) to (7)) each having a spiro bond. However, when Y is a group represented by the general formula (7), a case where X in the general formula (1) has a backbone structure selected from a group consisting of pyrenylene backbone structure, chrysenylene backbone structure and phenanthlene backbone structure is excluded.

In the general formula (1), n represents an integer of 0 to 4, and preferably an integer of 1 or 2. Further, m represents an integer of 0 to 2, preferably an integer of 0 or 1.

However, when Sp in the general formula (1) is a spirobifluorenyl group, a case where X has a backbone structure selected from a group consisting of pyrenylene backbone structure, chrysenylene backbone structure and phenanthlene backbone structure is excluded.

In the general formula (1), it is preferable that Sp represented by the general formula (2) is a group having a spiro bond represented by a following general formula (3).

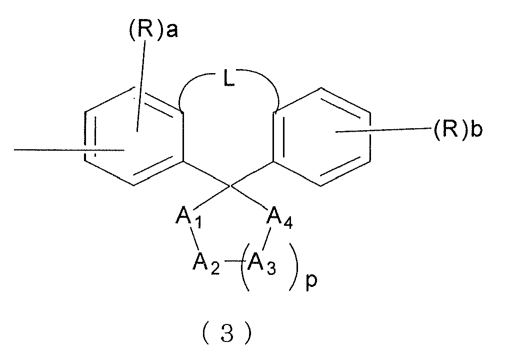

In the general formula (3), R, L, a and b each independently is the same as aforementioned about the general formula (2).

In the general formula (3), A₁ to A₄ each independently represents ―CR'R"― ,―SiR'R"―, ―O―, ―NR'― or ―CO―.

In the above description, R' and R" each independently represents a hydrogen atom, a substituted or unsubstituted aromatic group having 6 to 50 ring carbon atoms, a substituted or unsubstituted heterocyclic group having 5 to 50 ring atoms, or a substituted or unsubstituted alkyl group having 1 to 50 carbon atoms; and further, R' and R" may be the same with or different from each other, which may bond each other to form a ring structure.

In the general formula (3), p represents an integer of 1 to 10.

In the general formula (3), it is preferable that at least two adjacent component among A₁ to A₄ are represented by ―CR'R"― wherein R' and R" are the same as the above description, and that the adjacent R's, the adjacent R"s or both R' and R" may bond saturatedly or unsaturatedly forming a ring structure having 4 to 50 carbon atoms as a result.

Examples of the ring structure having 4 to 50 carbon atoms include a cycloalkane having 4 to 12 carbon atoms such as cyclobutane, cyclopentane, cyclohexane, adamantane, norbornane, etc.; a cycloalkene having 4 to 12 carbon atoms such as cyclobutene, cyclopentene, cyclohexene, cyclo heptene, cyclo octene, etc.; a cycloalkadiene having 6 to 12 carbon atoms such as cyclohexa diene, cyclohepta diene, cyclo octadiene, etc.; and an aromatic ring having 6 to 50 carbon atoms such as benzene, naphthalene, phenanthrene, anthracene, pyrene, chrysene, acenaphthylene, etc.

In the general formula (1), it is also preferable that Sp represented by the general formula (2) is a group shown by any one of the following general formulae (4) to (7):

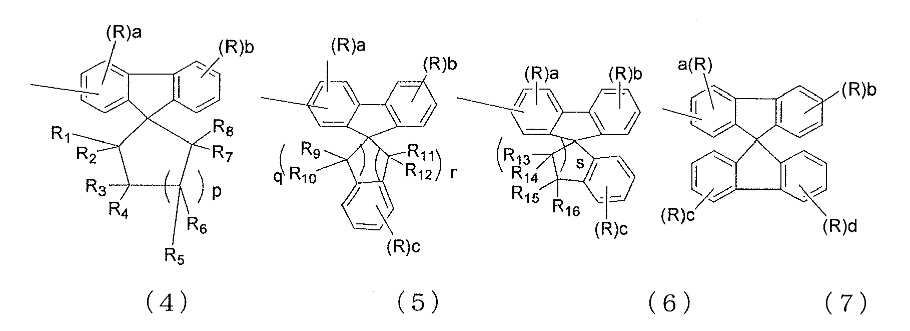

In the general formulae (4) to (7), as already described, R represents a substituted or unsubstituted aromatic group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, a carboxyl group, a halogen atom, a cyano group, a nitro group or a hydroxyl group.

In the general formulae (4) to (7), R₁ to R₁₆ each independently represents a hydrogen atom, a substituted or unsubstituted aromatic group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, a carboxyl group, a halogen atom, a cyano group, a nitro group or a hydroxyl group. At least two among R₁ to R₁₆ may bond each other to form a ring structure.

Further, specific examples of the aromatic group, the aromatic heterocyclic group, the alkyl group, the alkoxy group, the aralkyl group, the aryloxy group, the arylthio group and the alkoxycarbonyl group are the same as those already described in the foregoing explanation about R.

In the general formulae (4) to (7), a, b, c and d each represents an integer of 0 to 4, preferably an integer of 0 to 2.

In the general formulae (4) to (7), p, q, r and s each represents an integer of 1 to 10, preferably an integer of 1 to 5.

It is preferable that X in the general formula (1) is a group shown by any one of the following general formulae (8) to (25).

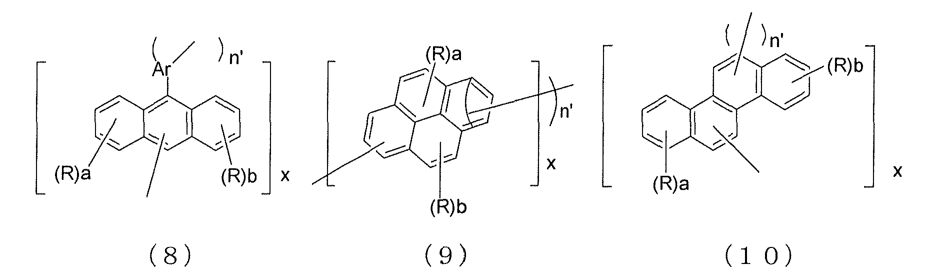

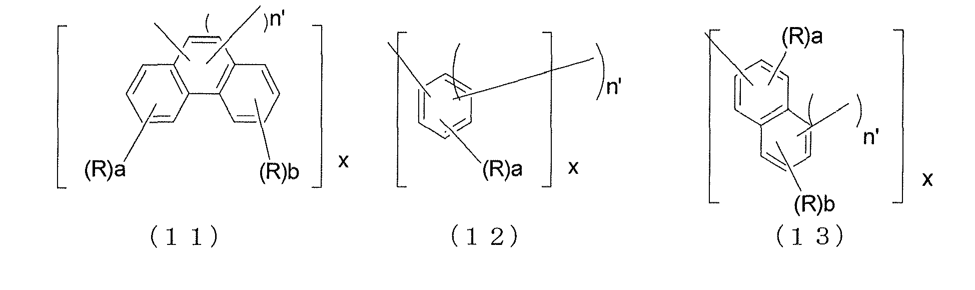

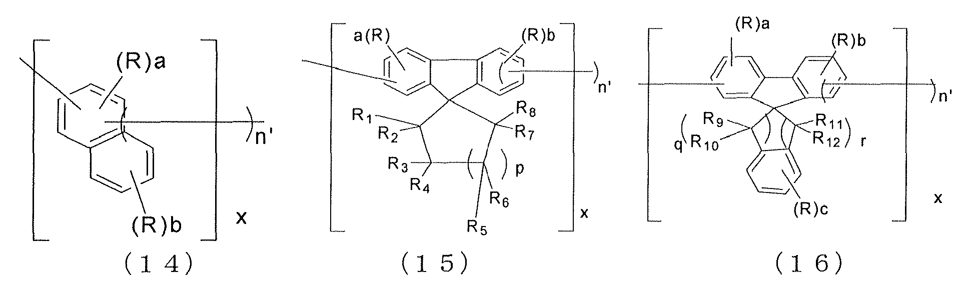

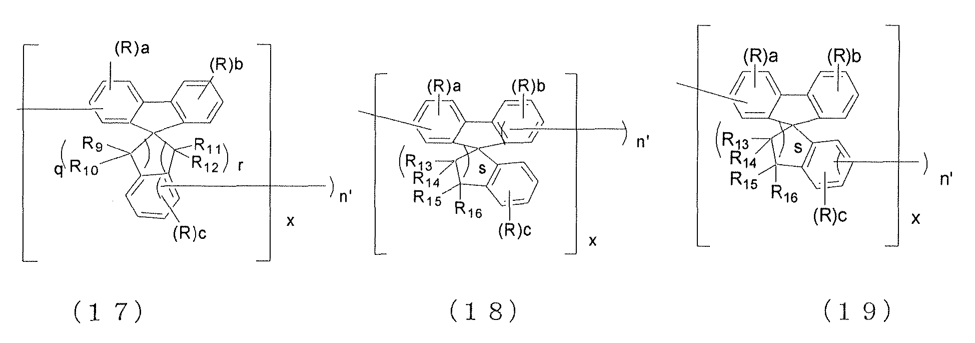

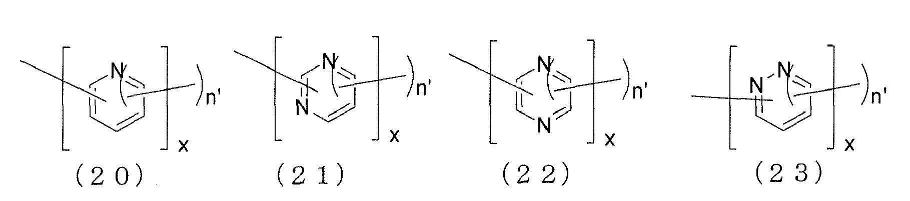

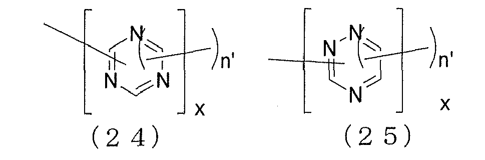

In the general formulae (8) to (25), R, R₁ to R₁₆, a to d and p to s are the same as the previous description.

In the general formulae (8) to (25), Ar represents a substituted or unsubstituted aromatic group having 6 to 50 ring carbon atoms, a substituted or unsubstituted aromatic heterocyclic group having 5 to 50 ring atoms, or a group made by combining plural of those preceding groups. However, Ar is not an anthracendiyl group or a polyanthracendiyl group.

Further, specific examples of the aromatic group, the aromatic heterocyclic group are the same as those already described in the foregoing explanation about R.

In the general formulae (8) to (25), n' represents an integer of 0 to 5, preferably an integer of 0 to 2.

In the general formulae (8) to (25), x represents an integer of 1 to 20, preferably an integer of 1 to 10.

However, when Sp in the general formula (1) is a group having a spiro bond shown by the general formula (7), a case where X is a group shown by any one of the general formulae (9) to (11) is excluded.

Furthermore, X may be a group formed by combining plural of groups shown by any of the general formulae (8) to (25) together. The term "combining" is defined as coupling 2 or more selected units by means of single bond(s) together. Examples include (9) - (12), (10)-(12), (11) - (12) , (12)―(13), (12)―(14), (12)―(15), (12)―(16), (12)―(17), (12)―(18), (12)―(19), (12)―(20), (12)―(21), (12)―(22), (12)―(23), (12)―(24), (12)― (25), (9)―(12)―(9), (10)―(12)―(10), (11)―(12)―(11), (13)―(12)―(13), (14)―(12)―(14), (15)―(12)―(15), (16)―(12)―(16), (17)―(12)―(17), (18)-(12)―(18), (19)―(12)―(19), (20)―(12)―(20), (21)―(12)―(21), (22)―(12)-(22), (23)―(12)―(23), (24)―(12)―(24), (25)―(12)―(25), (8)―(15) , (9)― (15), (10)―(15), (11)―(15) (13)―(15), (14)―(15), (16)―(15), (17)―(15), (18)―(15), (19)―(15), (8)―(15)―(8), (9)―(15)―(9), (10)―(15)―(10), (11)― (15)―(11), (13)―(15)―(13), (14)―(15)―(14), (16)―(15)―(16), (17)―(15)― (17), (18)―(15)―(18), (19)―(15)―(19), (8)―(16), (9)―(16), (10)―(16), (11) ―(16), (13)―(16), (14)―(16), (17)―(16), (18)―(16), (19)―(16), (8)―(16)― (8), (9)―(16)―(9), (10)―(16)―(10), (11)―(16)―(11), (13)―(16)―(13), (14) ―(16)―(14), (17) ― (16) ― (17), (18) ― (16) ― (18), (19) ― (16) ― (19), etc. However the combination is not limited to those described above.

Specific examples of the compound having a spiro bond represented by the general formula (1) of the present invention include the following compounds, though not limited thereto.

Additionally, each numbers described in ring structure shows number of a member to compose the ring structure.

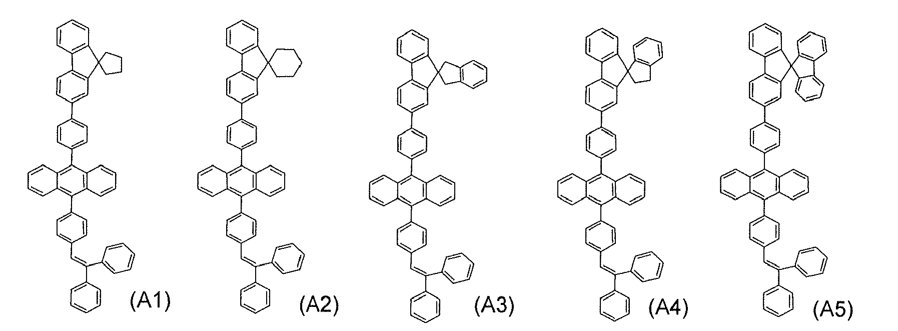

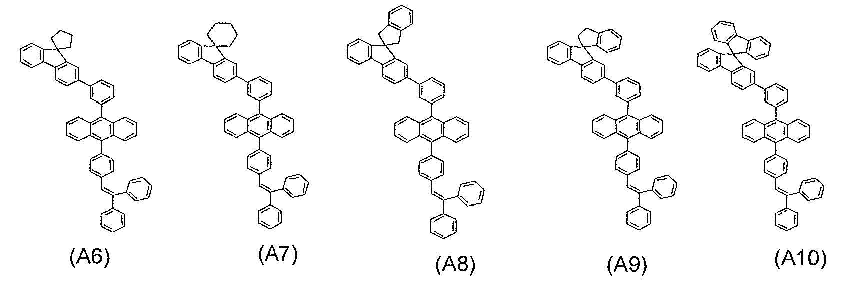

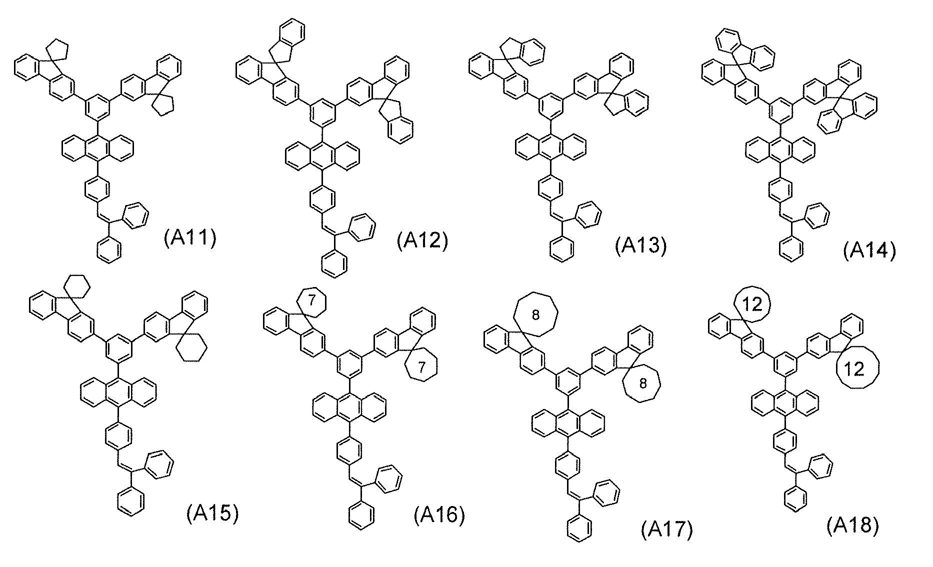

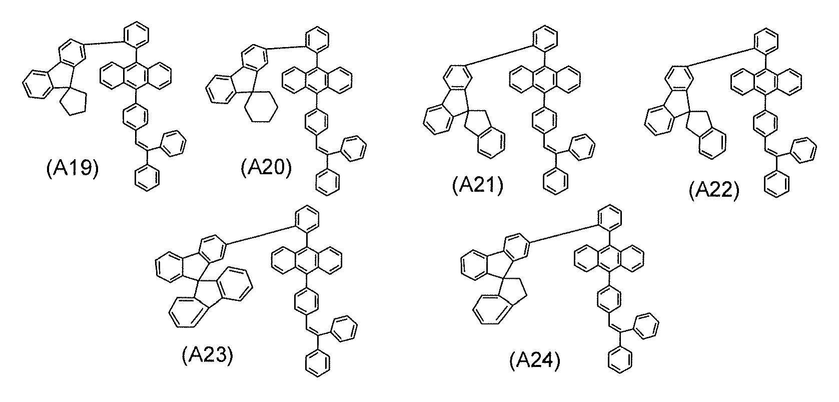

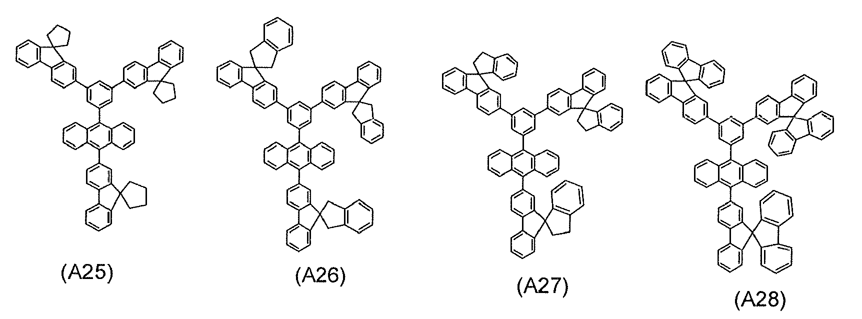

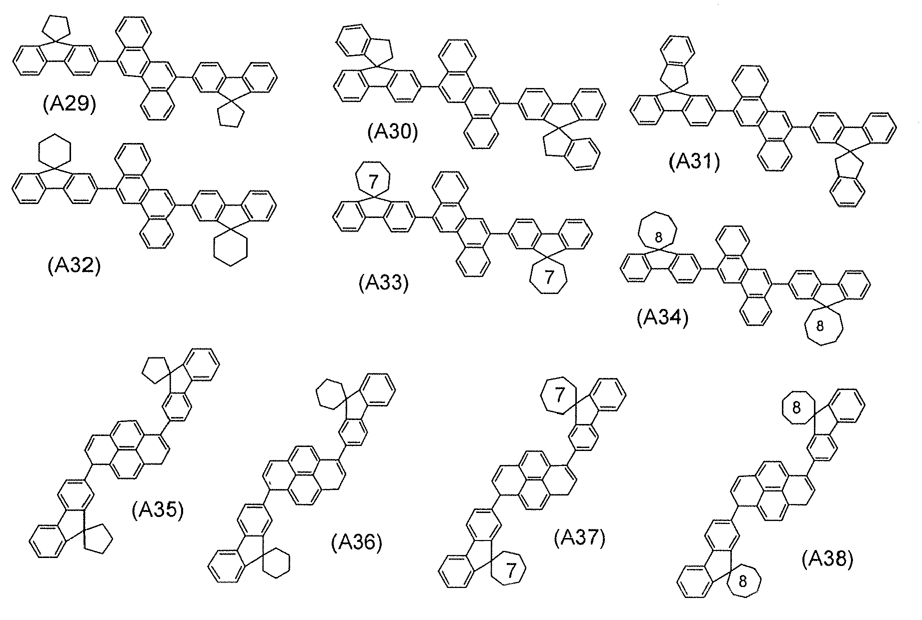

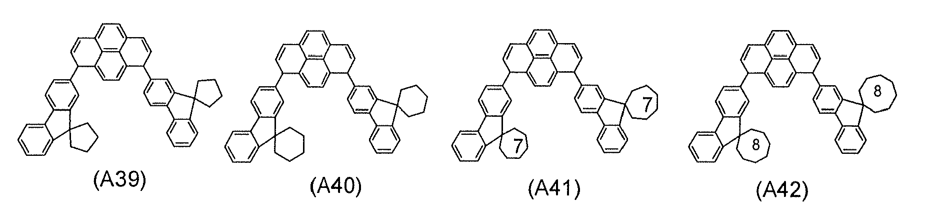

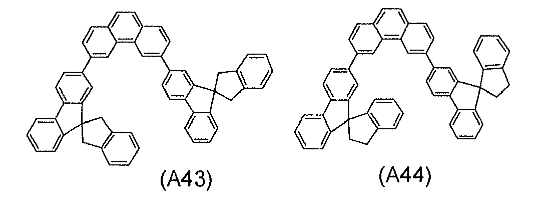

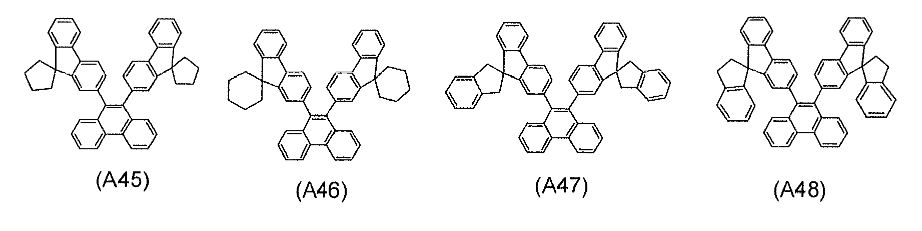

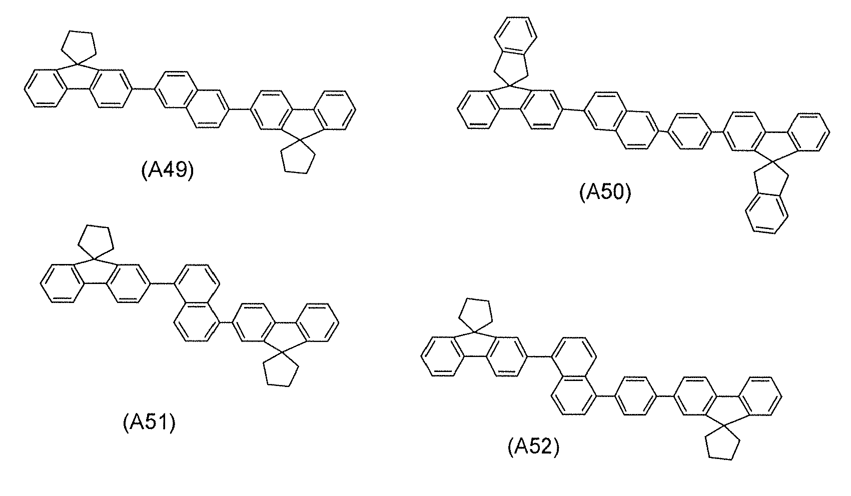

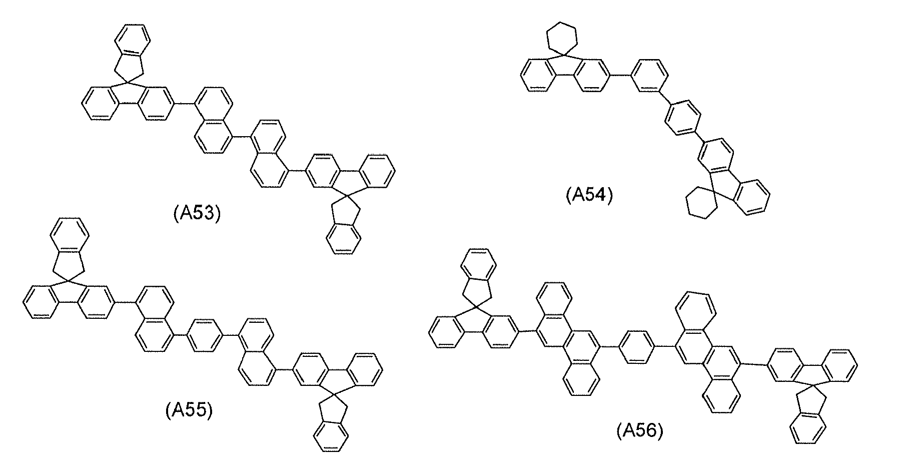

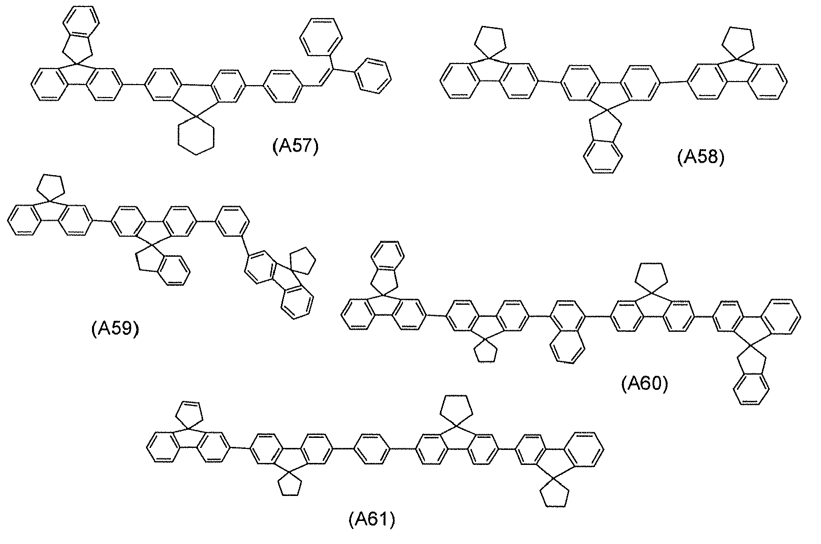

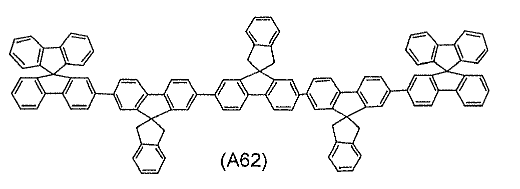

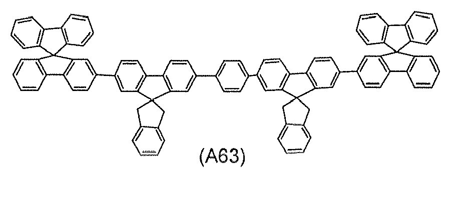

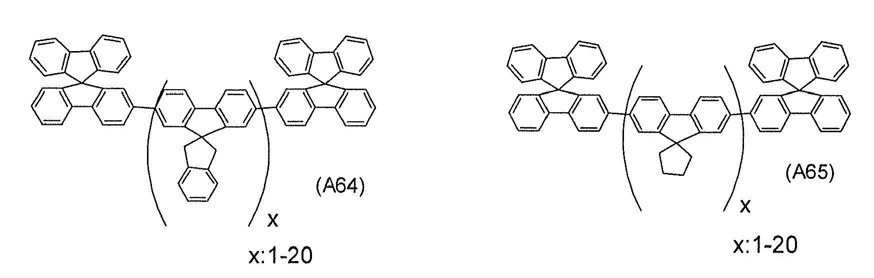

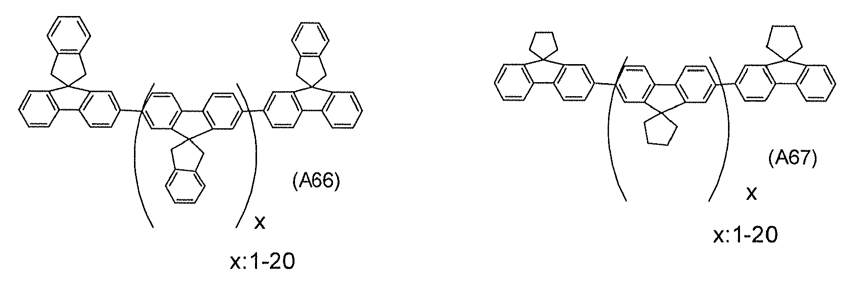

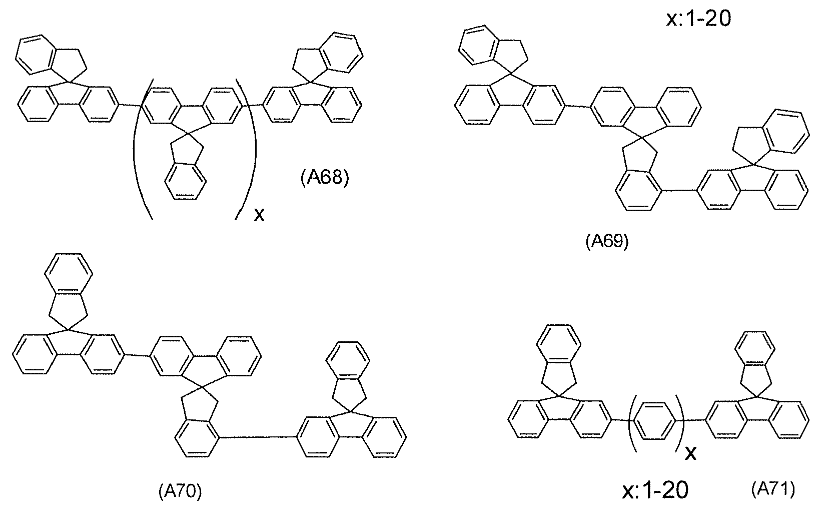

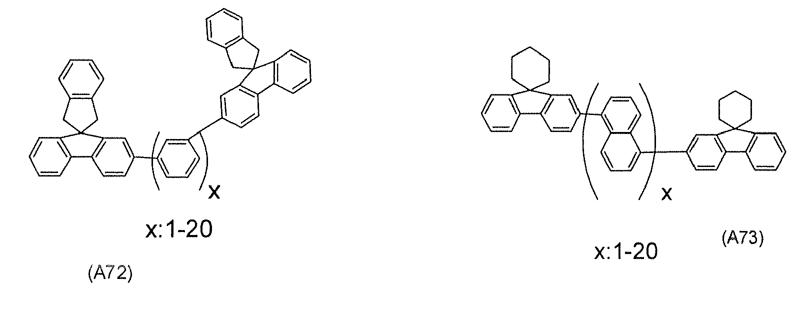

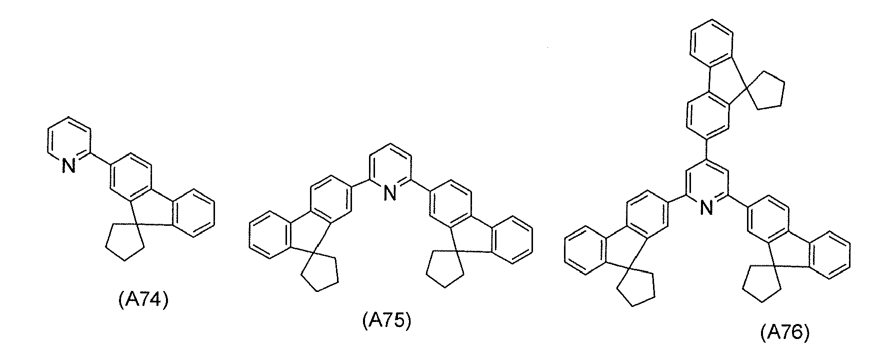

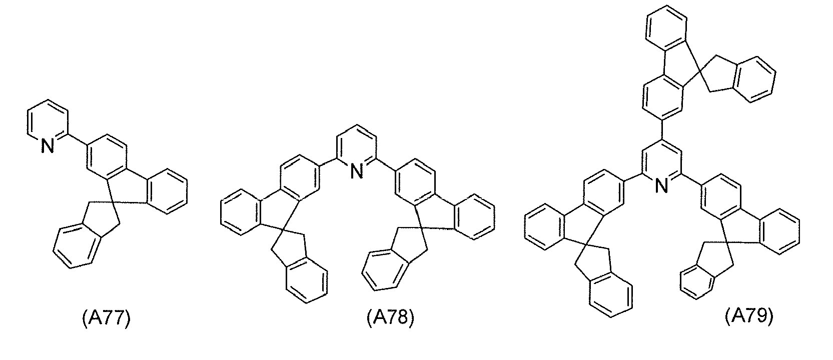

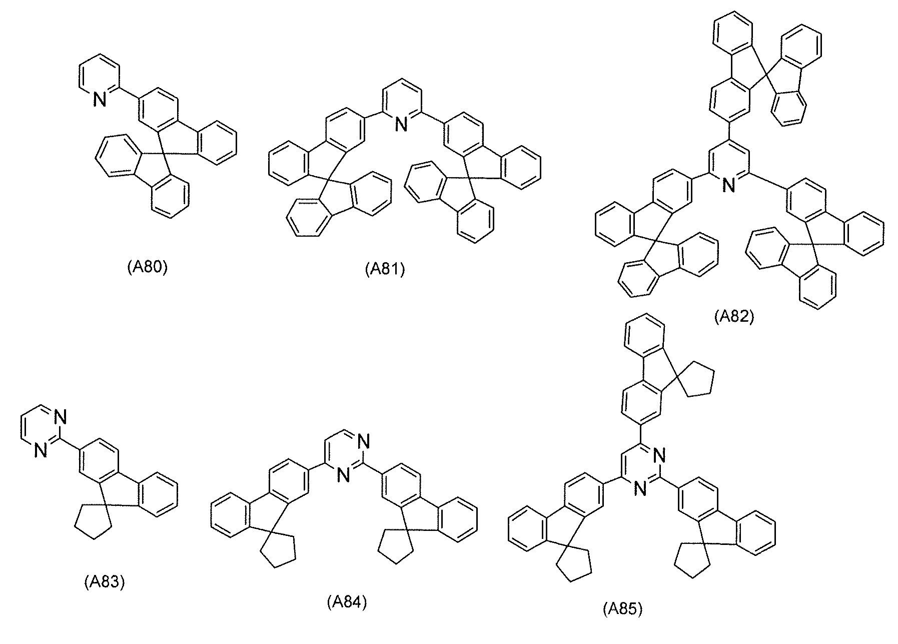

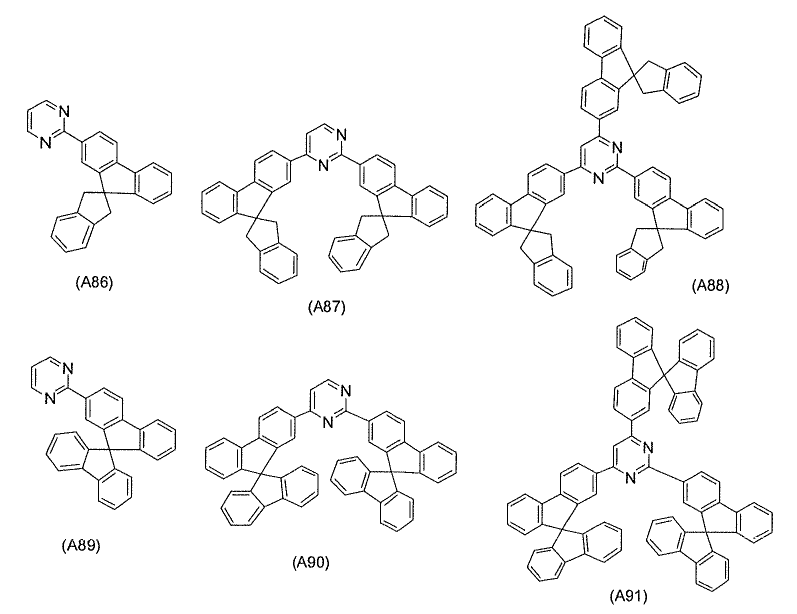

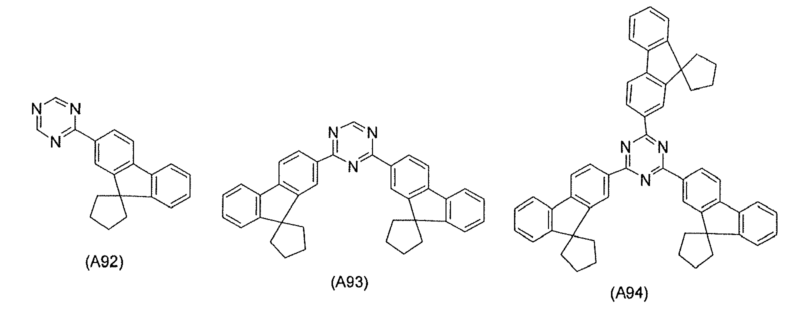

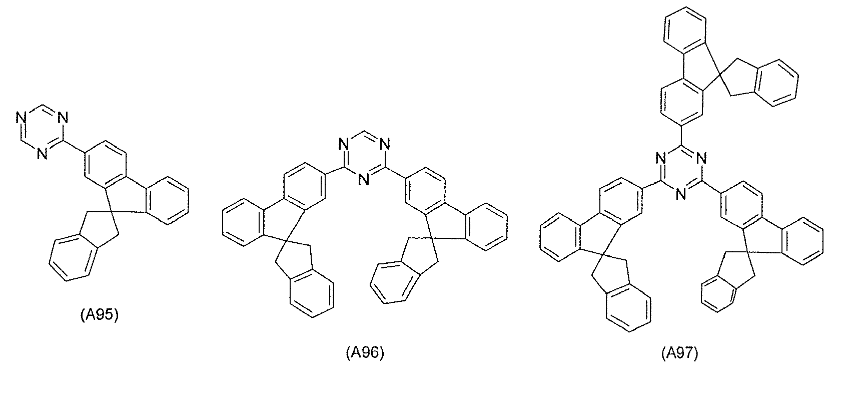

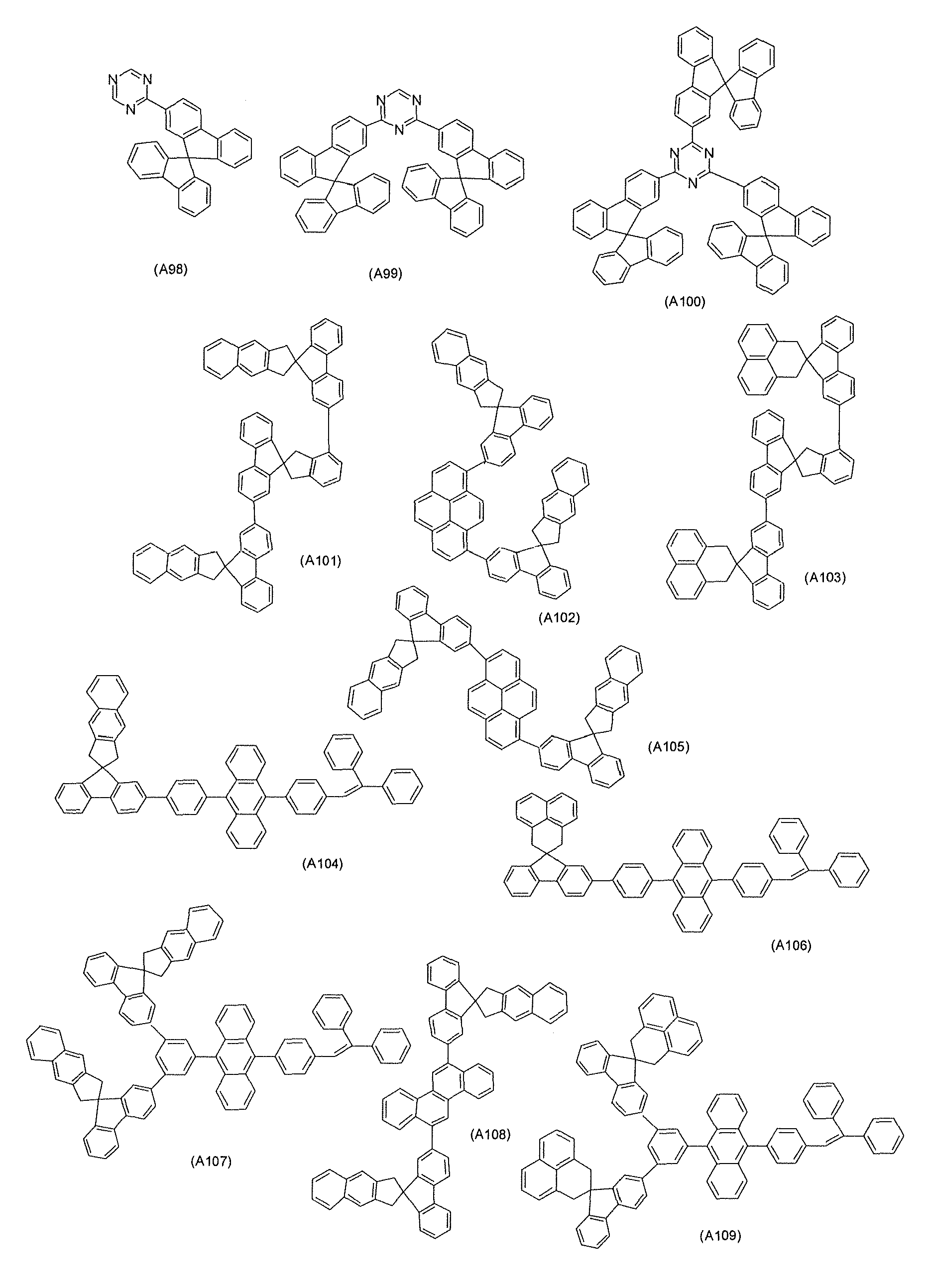

Following is a description regarding a device structure about the organic EL device of the present invention.

The present invention provides an organic EL device which comprises at least one organic thin film layer sandwiched between a pair of electrode consisting of an anode and a cathode, wherein the organic thin film layer comprises the foregoing compound having a spiro bond.

Typical examples of the construction of the organic EL device of the present invention include:
(1) An anode / a light emitting layer / a cathode;
(2) An anode / a hole injecting layer / a light emitting layer / a cathode;
(3) An anode / a light emitting layer / an electron injecting layer / a cathode;
(4) An anode / a hole injecting layer / a light emitting layer / an electron injecting layer / a cathode;
(5) An anode / an organic semiconductor layer / a light emitting layer / a cathode;
(6) An anode / an organic semiconductor layer / an electron barrier layer /a light emitting layer / a cathode;
(7) An anode / an organic semiconductor layer / a light emitting layer / an adhesion improving layer / a cathode;
(8) An anode / a hole injecting layer / a hole transporting layer / a light emitting layer / an electron injecting layer / a cathode;
(9) An anode / an insulating layer / a light emitting layer / an insulating layer / a cathode;
(10) An anode / an inorganic semiconductor layer / an insulating layer / a light emitting layer / an insulating layer / a cathode;
(11) An anode / an organic semiconductor layer / an insulating layer / a light emitting layer / an insulating layer / a cathode;
(12) An anode / an insulating layer / a hole injecting layer / a hole transporting layer / a light emitting layer / an insulating layer / a cathode; and
(13) An anode / an insulating layer / a hole injecting layer / a hole transporting layer / a light emitting layer / an electron injecting layer / a cathode.

However, the construction of the organic EL device is not limited to those shown above as the examples.

It is preferable, though not limited to, for the organic EL device of the present invention to employ the compound having a spiro bond of the present invention as a constituting material for the light emitting layer. In a case where the compound having a spiro bond of the present invention is employed in the light emitting layer, the compound can be usually used with various organic materials employed for the organic EL device in combination. It is particularly preferable to employ an amine compound having a styryl group represented by a general formula (27) or an arylamine compound represented by a general formula (28) each as a dopant respectively.

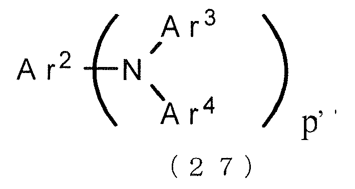

In the general formula (27), Ar² represents a group selected from among a phenyl group, a biphenyl group, a terphenyl group, a stilbene group and a distyryl aryl group; Ar³ and Ar⁴ each independently represents a hydrogen atom or an aromatic group having 6 to 20 ring carbon atoms; and each of Ar² to Ar⁴ may be substituted; p' represents an integer of 1 to 4; and more preferably, at least one of Ar³ and Ar⁴ is substituted with a styryl group.

In the preceding description, the aromatic group having 6 to 20 ring carbon atoms is preferably a phenyl group, a naphthyl group, an anthranil group, a phenanthryl group, a terphenyl group or so.

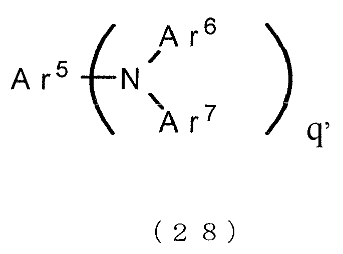

In the general formula (28), Ar⁵ to Ar⁷ each independently represents an aryl group having 5 to 40 ring carbon atoms that may be substituted; and q' represents an integer of 1 to 4.

In the preceding description, the aryl group having 5 to 40 ring carbon atoms is preferably a phenyl group, a naphthyl group, an anthranil group, a phenanthryl group, a pyrenyl group, a coronyl group, a biphenyl group, a terphenyl group, a pyrrolyl group, a furanyl group, a thiophenyl group, a benz thiophenyl group, an oxadiazolyl group, a diphenyl anthranil group, an indolyl group, a carbazolyl group, a pyridyl group, a benz quinolyl group, a fluoranthenyl group, an acenaphthofluoranthenyl group, a stilbene group or so. Additionally, the aryl group having 5 to 40 ring carbon atoms may be further substituted with a substituent, and preferable examples of the substituent include an alkyl group having 1 to 6 carbon atoms (an ethyl group, a methyl group, an i-propyl group, a n-propyl group, a s-butyl group, a t-butyl group, a pentyl group, a hexyl group, a cyclopentyl group, a cyclohexyl group, etc.), an alkoxy group having 1 to 6 carbon atoms (an ethoxy group, a methoxy group, an i-propoxy group, a n-propoxy group, a s- butoxy group, a t-butoxy group, a pentoxy group, a hexyloxy group, a cyclo pentoxy group, a cyclohexyl oxy group, etc.), an aryl group having 5 to 40 ring atoms, an amino group substituted with an aryl group having 5 to 40 ring atoms, an ester group which has an aryl group having 5 to 40 ring atoms, an ester group which has an alkyl group having 1 to 6 carbon atoms, a cyano group, a nitro group, and a halogen atom (a chlorine atom, a bromine atom, an iodine atom, etc.).

In general, the organic EL device is produced on a substrate which transmits light. It is preferable that the substrate which transmits light has a transmittance of light of 50% or greater in the visible region of 400 to 700 nm. It is also preferable that a flat and smooth substrate is employed.

As the substrate which transmits light, for example, glass sheet and synthetic resin sheet are advantageously employed. Specific examples of the glass sheet include soda ash glass, glass containing barium and strontium, lead glass, aluminosilicate glass, borosilicate glass, barium borosilicate glass and quartz. Specific examples of the synthetic resin sheet include sheet made of polycarbonate resins, acrylic resins, polyethylene terephthalate resins, polyether sulfide resins and polysulfone resins.

The anode in the organic EL device of the present invention covers a role of injecting holes into a hole transport layer or into a light emitting layer, and it is effective that the anode has a work function of 4.5 eV or greater. Specific examples of the material for the anode include indium tin oxide alloy (ITO), tin oxide (NESA), gold, silver, platinum, copper, etc. With regard to the cathode, its material preferably has a small work function with the aim of injecting electrons into an electron transport layer or into a light emitting layer.

The anode can be prepared by forming a thin film of the electrode material described above in accordance with a process such as a vapor deposition process or a sputtering process.

When the light emitted from the light emitting layer is observed through the anode, it is preferable that the anode has a transmittance of the emitted light greater than 10 %. It is also preferable that the sheet resistivity of the anode is several hundred Ω/□ or smaller. The thickness of the anode is, in general, selected in the range of from 10 nm to 1 *µ*m and preferably in the range of from 10 to 200 nm.

In the organic EL device of the present invention, the light emitting layer has the following functions:
(1) The injecting function: the function of injecting holes from the anode or the hole injecting layer and injecting electrons from the cathode or the electron injecting layer when an electric field is applied;
(2) The transporting function: the function of transporting injected charges (electrons and holes) by the force of the electric field; and
(3) The light emitting function: the function of providing the field for recombination of electrons and holes and leading the recombination to the emission of light.

Although there may be a difference between the capability of the holes being injected and the capability of the electrons being injected, and although there may be a grade about the transporting function expressed by mobility of the holes and the electrons, it is preferable to move charges of either ones.

As the process for forming the light emitting layer, a well known process such as the vapor deposition process, the spin coating process and the LB process can be employed.

In the present invention, any well known light emitting material other than the compound having a spiro bond of the present invention may be optionally contained in the light emitting layer; or a light emitting layer containing other well known light emitting layer may be laminated with the light emitting layer containing the light emitting material of the present invention each in an extent of not obstructing to achieve the object of the present invention respectively.

In the present invention, the hole injecting layer and the hole transporting layer are layers which assist injection of holes into the light emitting layer and transport the holes to the light emitting zone. The layers exhibit a great mobility of holes and, in general, have an ionization energy as small as 5.5 eV or smaller. For the hole injecting layer and the hole transporting layer, a material which transports holes to the light emitting layer at a small strength of the electric field is preferable. A material which exhibits, for example, a mobility of holes of at least 10⁻⁴ cm²/V · sec under application of an electric field of from 10⁴ to 10⁶ V/cm is preferable.

When the compound of the present invention is employed in the hole transporting zone, the hole injecting layer or the hole transporting layer may be composed of only the compound of the present invention singly or may be composed of both the compound of the present invention and any other material in combination.

With regard to the material which may be employed for forming the hole injecting layer or the hole transporting layer in combination with the compound of the present invention, any material having the foregoing preferable properties is employed without particularly restricted, any arbitrary material selected from conventional material commonly used as a charge transporting material for the holes in photoconducting materials and well known material employed for the hole injecting layer in the EL device is usable. Examples include aromatic tertiary amines, hydrazone derivatives, carbazole derivatives, triazole derivatives, imidazole derivatives; and further include polyvinylcarbazole, polyethylene dihydroxy thiophene poly sulfonic acid (PEDOT-PSS), etc. Further examples include triazole derivatives (refer to United States Patent No. 3,112,197, etc.), oxadiazole derivatives (refer to United States Patent No. 3,189,447, etc.), imidazole derivatives (refer to Japanese Examined Patent KOKOKU No. Shou 37-16096, etc.), poly arylalkane derivatives (refer to United States Patent Nos. 3,615,402, 3,820,989 and 3,542,544, Japanese Examined Patent KOKOKU Nos. Shou 45-555 and Shou 51-10983, Japanese Unexamined Patent Application Laid-Open Nos. Shou 51-93224, Shou 55-17105, Shou 56-4148, Shou 55-108667, Shou 55-156953, Shou 56-36656, etc.), pyrazoline derivatives and pyrazolone derivatives (refer to U.S Patent Nos. 3,180,729 and 4,278,746, Japanese Unexamined Application Patent Laid-Open Nos. Shou 55-88064, Shou 55-88065, Shou 49-105537, Shou 55-51086, Shou 56-80051, Shou 56-88141, Shou 57-45545, Shou 54-112637, Shou 55-74546, etc.), phenylenediamine derivatives (refer to U.S Patent No. 3,615,404, Japanese Examined Patent KOKOKU Nos. Shou 51-10105, Shou 46-3712 and Shou 47-25336, Japanese Unexamined Patent Application Laid-Open Nos. Shou 54-53435, Shou 54-110536, Shou 54-119925, etc.), arylamine derivatives (refer to U.S Patent Nos. 3,567,450, 3,180,703, 3,240,597, 3,658,520, 4,232,103, 4,175,961 and 4,012,376, Japanese Examined Patent KOKOKU Nos. Shou 49-35702 and Shou 39-27577, Japanese Unexamined Patent Application Laid-Open Nos. Shou 55-144250, Shou 56-119132 and Shou 56-22437, West German Patent No. 1,110,518, etc.), chalcone derivatives which is substituted with amino group (refer to U.S Patent No. 3,526,501, etc.), oxazole derivatives (disclosed in U.S Patent No. 3,257,203, etc.), styryl anthracene derivatives (refer to Japanese Unexamine Patent Application Laid-Open No. Shou 56-46234, etc.), fluorenone derivatives (refer to Japanese Unexamined Patent Application Laid-Open No. Shou 54-110837, etc.), hydrazone derivatives (refer to U.S Patent Nos. 3,717,462, Japanese Unexamined Patent Application Laid-Open Nos. Shou 54-59143, Shou 55-52063, Shou 55-52064, Shou 55-46760, Shou 55-85495, Shou 57-11350, Shou 57-148749, Hei 2-311591, etc.), stilbene derivatives (refer to Japanese Unexamined Patent Application Laid-Open Nos. Shou 61-210363, Shou 61-228451, Shou 61-14642, Shou 61-72255, Shou 62-47646, Shou 62-36674 , Shou 62-10652, Shou 62-30255, Shou 60-93455, Shou 60-94462, Shou 60-174749, Shou 60-175052, etc.), silazane derivatives (U.S Patent No. 4,950,950), polysilane-based copolymers (Japanese Unexamined Patent Application Laid-Open No. Hei 2-204996), aniline-based copolymers (Japanese Unexamined Patent Application Laid-Open No. Hei 2-282263), an electroconductive polymer oligomer which is disclosed in Japanese Unexamined Patent Application Laid-Open No Hei 1-211399 (particularly, thiophene oligomer), etc.

With regard to the material of the hole injecting layer, the above materials are also employable, however, porphyrin compounds, aromatic tertiary amine compounds and styryl amine compounds (refer to U.S Patent No. 4,127,412, Japanese Unexamined Patent Application Laid-Open Nos. Shou 53-27033, Shou 54-58445, Shou 54-149634, Shou 54-64299, Shou 55-79450, Shou 55-144250, Shou 56-119132, Shou 61-295558, Shou 61-98353, Shou 63-295695, etc.) are preferable and the aromatic tertiary amine compounds are particularly preferable. Further examples include, for example, 4,4'-bis (N-(1-naphthyl)-N-phenylamino) biphenyl (abbreviated as NPD hereunder) having 2 fused aromatic rings in its molecular described in U.S Patent No. 5,061,569, 4,4',4"-tris (N-(3-methylphenyl)-N-phenylamino) triphenyl amine (abbreviated as MTDATA hereunder) made by connecting three triphenyl amine units to form a star burst type, etc.

Further, except the above-mentioned aromatic dimethylidene-based compound described as a material for the light emitting layer, inorganic compound such as p-type silicon, p-type silicon carbide or so is employable as the material for the hole injecting layer.

To form the hole injecting layer or the hole transporting layer, a thin film may be formed from the material for the hole injecting layer or the hole transporting layer, respectively, in accordance with a well known process such as the vacuum vapor deposition process, the spin coating process, the casting process and the LB process. Although the thickness of the hole injecting layer and the hole transporting layer is not particularly limited, the thickness is usually from 5 nm to 5 *µ* m. It is preferable that the hole injecting layer or the hole transporting layer comprises the compound of the present invention in the hole transporting zone. Further, the hole injecting layer or the hole transporting layer may be composed of single layer comprising one or more kind of those materials or may be laminated with a hole injecting layer or a hole transporting layer each comprising another kind of compound respectively.

In the organic EL device of the present invention, the organic semiconductor layer assists to inject the holes or to inject the electrons into the light emitting layer, and it is preferable for the organic semiconductor layer to have a electric conductivity of 10⁻¹⁰ S/cm or greater. With regard to a material for the organic semiconductor layer, electroconductive oligomers such as an oligomer having thiophene, an oligomer having arylamine disclosed in Japanese Unexamined Patent Application Laid-Open No. 8-193191 and so on, electroconductive dendrimers such as a dendrimer having an arylamine dendrimer and so on are employable.

The electron injection layer in the organic EL device of the present invention is a layer which assists injection of electrons into the light emitting layer and exhibits a great mobility of electrons. Among the electron injecting layers, an adhesion improving layer is a layer made of a material exhibiting excellent adhesion with the cathode. As the material for the electron injecting layer, 8-hydroxyquinoline, metal complexes of derivatives thereof and oxadiazole derivarives are preferable.

Examples of the 8-hydroxyquinoline and metal complexes of derivatives thereof include metal chelates of oxinoid compounds including chelates of oxine (in general, 8-quinolinol or 8-hydroxyquinoline). For example, tris(8-quinolinol)aluminum (Alq) can be employed as the electron injecting material.

Further, examples of the oxadiazole delivertives include an electron transfer compound shown as the following general formulae: 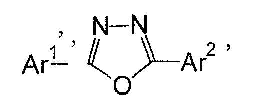 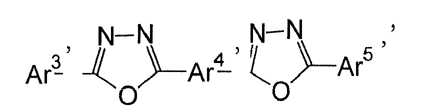 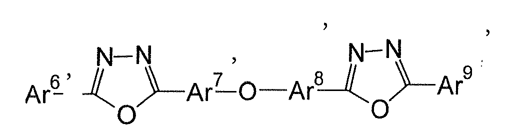
wherein Ar^{1'}, Ar^{2'}, Ar^{3'}, Ar^{5'}, Ar^{6'} and Ar^{9'} each independently represents a substituted or unsubstituted aryl group respectively, which may be the same with or different from each other; Ar^{4'}, Ar^{7'} and Ar^{8'} each independently represents a substituted or unsubstituted arylene group, which may be the same with or different from each other.

Examples of aryl group include a phenyl group, a biphenyl group, an anthranil group, a perilenyl group and a pyrenyl group. Further, examples of the arylene group include a phenylene group, a naphthylene group, a biphenylene group, an anthranylene group, a perilenylene group, a pyrenylene group, etc. Furthermore, examples of the substituent include an alkyl group having 1 to 10 carbon atoms, an alkoxy group or a cyano group each having 1 to 10 carbon atoms respectively, etc. With regard to the electron transfer compound, those compounds having a thin film forming capability are preferable.

Specific examples of the electron transfer compounds are shown below:

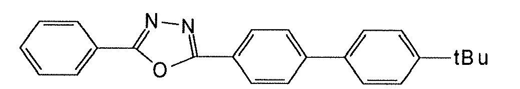

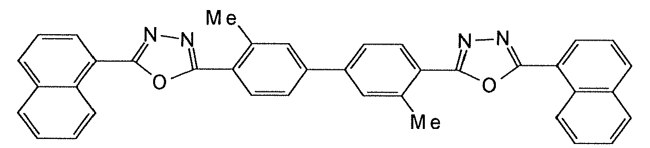

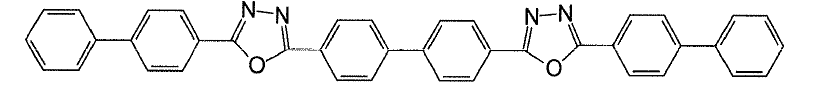

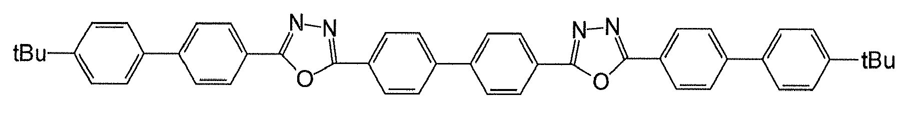

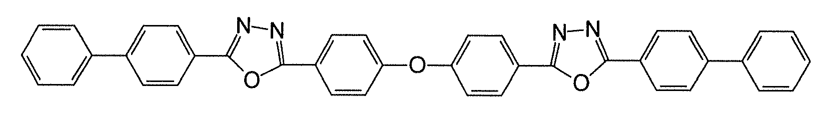

In the present invention, it is preferable that a reductive dopant is added in either the electron transporting zone or an interfacial zone between the cathode and the organic layer. The reductive dopant used in the present invention is defined as a substance which reduces the electron transporting compound. Examples of the reductive dopant include at least one compound selected from alkali metals, alkali metallic complexes, alkali metal compounds, alkaline earth metals, alkaline earth metallic complexes, alkaline earth metal compounds, rare earth metals, rare earth metallic complexes and rare earth metal compounds. Examples of the alkali metal compound, the alkaline earth metal compound and the rare earth metal compound described above include oxides and halides of the respective metals.

Examples of the preferable reductive dopant include at least one alkali metal selected from a group consisting of Li (the work function: 2.93 ev), Na (the work function: 2.36 eV), K (the work function: 2.28 eV), Rb (the work function: 2.16 eV) and Cs (the work function: 1.95 eV) or at least one alkaline earth metals selected from a group consisting of Ca (the work function: 2.9eV), Sr (the work function: 2.0 to 2.5 eV) and Ba (the work function: 2.52eV); whose work function of 3.0 eV or smaller is particularly preferable.

In the organic EL device of the present invention, an electron injecting layer formed with an insulating material or a semiconductor may be further sandwiched between the cathode and the organic thin film layer. The electron injecting layer effectively prevents leak in the electric current and improves the electron injecting capability.

It is preferable that at least one metal compound selected from the group consisting of alkali metal chalcogenides, alkaline earth metal chalcogenides, alkali metal halides and alkaline earth metal halides is used as the insulating material. It is preferable that the electron injecting layer is constituted with the above alkali metal chalcogenide since the electron injecting property can be improved. Preferable examples of the alkali metal chalcogenide include Li₂O, LiO, Na₂S, Na₂Se and NaO. Preferable examples of the alkaline earth metal chalcogenide include CaO, BaO, SrO, BeO, BaS and CaSe. Preferable examples of the alkali metal halide include LiF, NaF, KF, LiCl, KCl and NaCl. Preferable examples of the alkaline earth metal halide include fluorides such as CaF₂, BaF₂, SrF₂, MgF₂ and BeF₂ and halides other than the fluorides.

Examples of the semiconductor constituting the electron transporting layer include oxides, nitrides and oxide nitrides containing at least one element selected from Ba, Ca, Sr, Yb, Al, Ga, In, Li, Na, Cd, Mg, Si, Ta, Sb and Zn, which are used singly or in combination of two or more. It is preferable that the inorganic compound constituting the electron transporting layer is in the form of a fine crystalline or amorphous insulating thin film. When the electron transporting layer is constituted with the above insulating thin film, a more uniform thin film can be formed and defective pixels such as dark spots can be decreased. Examples of the inorganic compound include the alkali metal chalcogenides, the alkaline earth metal chalcogenides, the alkali metal halides and the alkaline earth metal halides which are described above.

As the cathode for the organic EL device of the present invention, an electrode substance such as metal, alloy, electroconductive compound and those mixture having a small work function (4 eV or smaller) is employed. Examples of the electrode substance include potassium, sodium- potassium alloy, magnesium, lithium, magnesium-silver alloy, aluminum / aluminum oxide, aluminum-lithium alloy, indium, rare earth metal, etc.

The cathode can be prepared by forming a thin film of the electrode material described above in accordance with a process such as the vapor deposition process and the sputtering process. When the light emitted from the light emitting layer is observed through the cathode, it is preferable that the cathode has a transmittance of the emitted light greater than 10 %. It is also preferable that the sheet resistivity of the cathode is several hundred Ω/□ or smaller. The thickness of the cathode is, in general, selected in the range of from 10 nm to 1 *µ*m and preferably in the range of from 50 to 200 nm.

In general, an organic EL device tends to form defects in pixels due to leak and short circuit since an electric field is applied to ultra-thin films. To prevent the formation of the defects, a layer of an insulating thin film may be inserted between the pair of electrodes.

Examples of the material employed for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide and vanadium oxide. Mixtures and laminates of the above compounds can also be employed.

The process for forming the layers in the organic EL device of the present invention is not particularly limited. A conventional process such as the vacuum vapor deposition process and the spin coating process can be used. The organic thin film layer comprising the compound having a spiro bond represented by the foregoing general formula (1) used in the organic EL device of the present invention can be formed in accordance with the vacuum vapor deposition process, the molecular beam epitaxy process (the MBE process) or, using a solution prepared by dissolving the compound into a solvent, in accordance with a conventional coating process such as the dipping process, the spin coating process, the casting process, the bar coating process and the roller coating process.

The thickness of each layer in the organic thin film layer in the organic EL device of the present invention is not particularly limited. In general, an excessively thin layer tends to have defects such as pin holes, and an excessively thick layer requires a high applied voltage results in decreasing the efficiency. Therefore, a thickness within the range of several nanometers to 1 *µ* m is preferable.

The organic EL device which can be produced as described above emits light when a direct voltage of 5 to 40 V is applied in the condition that the anode is connected to a positive electrode (+) and the cathode is connected to a negative electrode (-). When the connection is reversed, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be employed.

The compound having a spiro bond of the present invention has a favorable solubility to organic solvents because of its spiro-atom. Accordingly, the compound is favorably used for a fabrication of the organic EL device by means of a wet process, and even in a case where the compound of the present invention has too high molecular weight to easily form a thin film by vacuum deposition method, it is easy to form the thin film by means of any application method such as the dipping process, the spin coating process, the casting process, the bar coating process, the roller coating process, etc. The material for forming luminous coated film of the present invention essentially consists of an organic solvent solution comprising the compound having a spiro bond.

The material for forming a luminous coated film is defined as a material for providing an organic compound layer relating to light emission, specifically a light emitting layer, hole injecting (transporting) layer, electron injecting (transporting) layer, and so on by means of forming a coated film in the organic EL device.

Examples of the organic solvent used for dissolving the compound having a spiro bond of the present invention include, halogen-based hydrocarbon solvent such as dichloro-methane, dichloroethane, chloroform, tetrachloromethane, tetrachloro ethane, trichloroethane, chlorobenzene, dichlorobenzene, chlorotoluene, etc.; ether-based solvent such as dibutyl ether, tetrahydrofuran, dioxane, anisole, etc.; alcohol-based solvent such as methanol, ethanol, propanol, butanol, pentanol, hexanol, cyclohexanol, methyl cellosolve, ethylcellosolve, ethylene glycol, etc.; hydrocarbon-based solvent such as benzene, toluene, xylene, ethyl benzene, hexane, octane, decane, etc.; ester-based solvent such as ethyl acetate, butyl acetate, amyl acetate, etc. Among those, halogen-based hydrocarbon solvent, hydrocarbon-based solvent and ether-based solvent are preferable. Further, the solvent may be used alone, or in combination of two or more kind thereof. Additionally, the employable solvent is not limited to the above examples.

Still further, a dopant may be optionally dissolved in advance, into the solution of the material for forming the luminous coated film of the present invention. The amine compound shown by the foregoing general formulae (27) and (28) may be employable as the dopant. Moreover, other various additives may be dissolved in advance, if necessary.

This invention will be described in further detail with reference to Examples, which does not limit the scope of this invention.

### Example 1 (Synthesis of Compound (A11))

The route for synthesis of Compound (A11) is shown as the following. 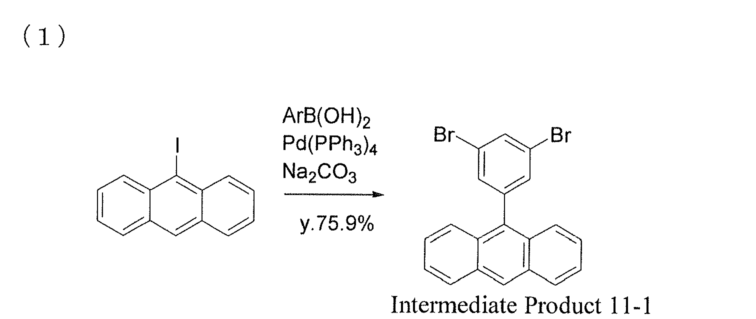 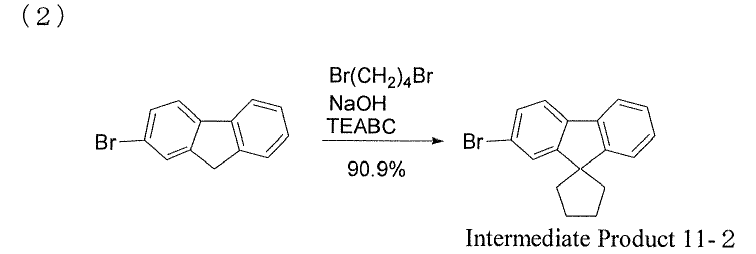 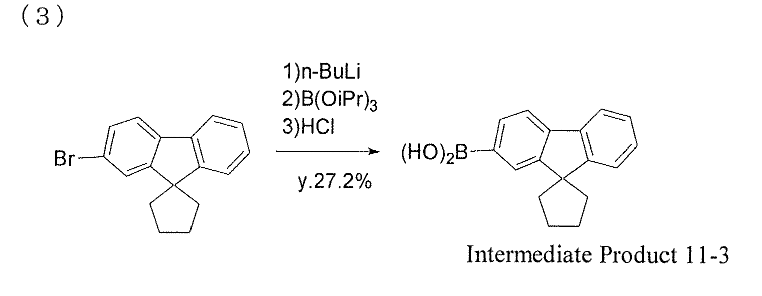 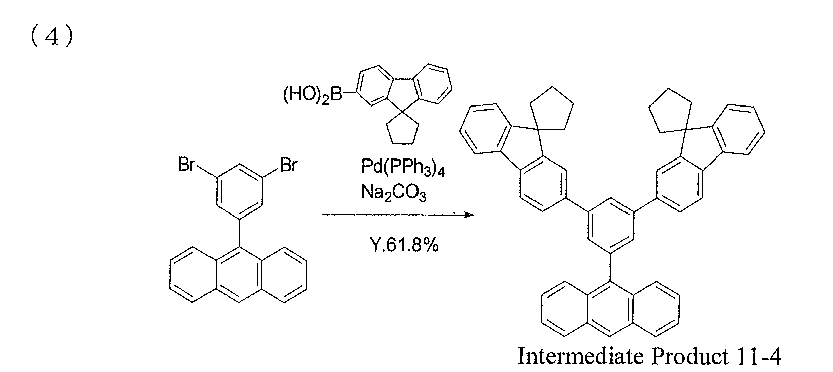 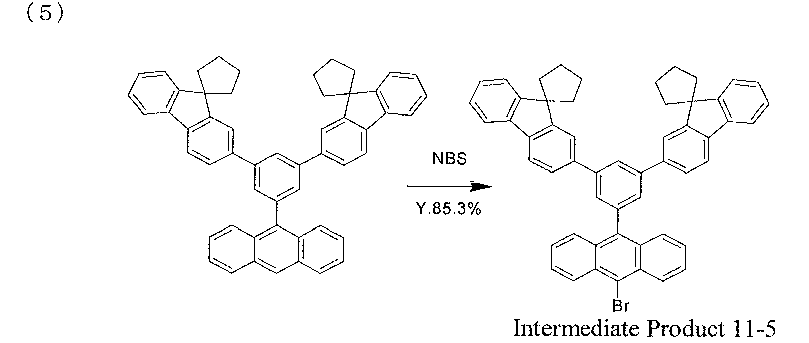 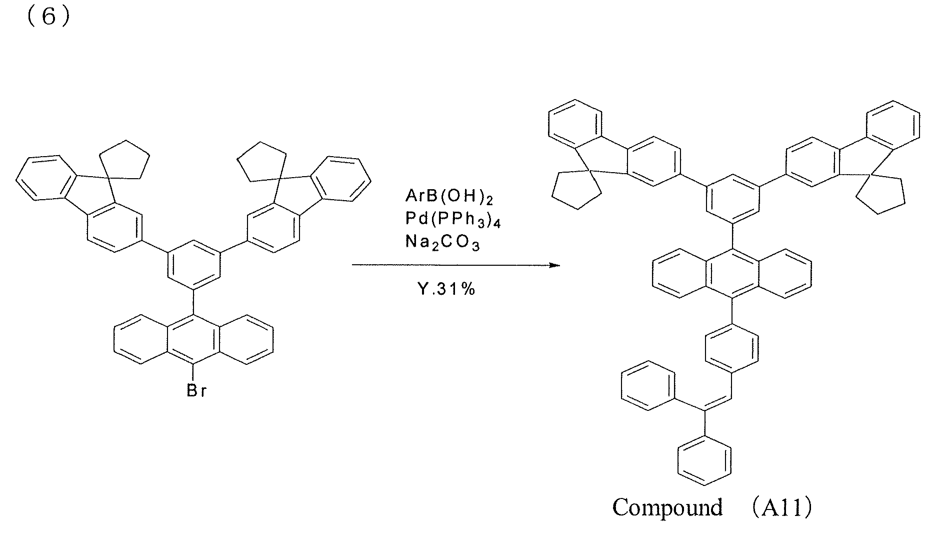
(1) Synthesis of Intermediate Product 11-1
   Into a three neck flask, 3,5-dibromobenzene-1-boronic acid (5.0 g, 17.9 mmol), 9-iodo anthracene (6.53 g, 21.5 mmol) and Pd(PPh₃)₄ (0.62 g, 0.54 mmol) are placed and the atmosphere in the flask was replaced with argon gas. An aqueous solution (27 milliliter) of toluene (50 milliliter) and sodium carbonate (5.69 g, 53.7 mmol) was added to the resultant solution and then, it was refluxed under heating for 8 hours. The resultant reaction solution was extracted with toluene, followed by vacuum concentration. The solid obtained after the extraction and the vacuum concentration was refined with silica gel column chromatography (dissolution solvent: methylene chloride) and as a result, Intermediate Product 11-1 was obtained.
   Produced Amount: 5.60g, Yield: 75.9 %
(2) Synthesis of Intermediate Product 11-2
   Into a three neck flask with a capacity of 100 milliliter and already replaced with argon gas, 2-bromo fluorene (10 g, 40.8 mmol), dimethylsulfoxide (15 milliliter) and benzyltriethylammonium chloride (0.19 g, 0.82 mmol) were placed and further, an aqueous solution (6.5 milliliter) of sodium hydroxide (50 % by weight) was dripped while stirring into the resultant solution. Subsequently, 1,4-dibromobutane (8.81 g, 40.8 mmol) was added and the resultant solution was stirred for 5 hours. Further, water (100 milliliter) and toluene (100 milliliter) were added to the resultant reaction solution and an organic layer was separated. The organic layer was dried with the use of anhydride magnesium sulfide, vacuum concentrated by means of an evaporator and as a result, Intermediate Product 11-2 was obtained. Measured value of 90MHz ¹H-NMR is shown as the following:
   Produced Amount: 11.1 g, Yield: 90.9 %
   ¹H-NMR (CDCl₃): δ (ppm) 7.7-7.2 (m, 7H), 2.1 (s, 8H)
(3) Synthesis of Intermediate Product 11-3
   Intermediate Product 11-2 (5.0 g, 16.7 mmol), anhydrous toluene (10 milliliter) and anhydrous ether (40 milliliter) were placed into a flask with a capacity of 300 milliliter and already replaced with argon gas, and further, after cooling the resultant solution down to -60 °C, a hexane solution (12.6 milliliter) of n-butyllithium 1.59 M was poured into the cooled solution. The resultant reaction solution was stirred at the temperature of ―20 °C for 1 hour and further, the solution was cooled down to ―60 °C, and then, adding anhydrous ether solution (40 milliliter) of triisopropyl boride (6.28 g, 33.4 mmol), the resultant solution was stirred for 1 hour. The temperature of the reaction solution was elevated little by little up to the room temperature, and the reaction solution was stood alone for a night. After adding 2N hydrochloric acid (100 milliliter) to the reacted solution, and after stirring the solution at room temperature for 1 hour, an organic layer was separated. The organic layer was dried with the use of anhydride magnesium sulfide, and then, it was vacuum concentrated by means of an evaporator. By washing the resultant solid with the used of n-hexane, Intermediate Product 11-3 was obtained. The result of the measurement in accordance with 90MHz ¹H-NMR is shown as the following:
   Produced Amount: 1.20 g, Yield: 27.2 %
   ¹H-NMR (CDCl₃): δ (ppm) 8.4-8.2 (m, 2H), 7.9-7.6 (m, 2H), 7.5-7.2 (m, 3H), 2.2 (br s, 8H):
(4) Synthesis of Intermediate Product 11-4
   Intermediate Product 11-1 (0.93 g, 2.27 mmol), Intermediate Product 11-3 (1.50 g, 5.68 mmol), Pd(PPh₃) 4 (0.16 g, 0.14 mmol), toluene (15 milliliter) and an aqueous solution (6.8 milliliter) of sodium carbonate (1.44 g, 13.6 mmol) were placed into a three neck flask already replaced with argon gas, and the resultant solution was heated at the temperature of 80 °C for 9 hours. Water (100 milliliter) and toluene (100 milliliter) were added to the resultant reaction solution, and an organic layer was separated, followed by drying with the use of anhydride magnesium sulfide. The dried organic layer was vacuum concentrated by means of an evaporator and the resultant oily substance was refined by means of a silica gel column chromatography (dissolution solvent: methylene chloride / hexane =1/3), and as a result, Intermediate Product 11-4 was obtained. The result of the measurement in accordance with 90MHz ¹H-NMR is shown as the following:
   Produced Amount: 0.97 g, Yield: 61.8 %
   ¹H-NMR (CDCl₃): δ (ppm) 8.6 (s, 1H), 8.2-7.8 (m, 5H), 7.7 (br s, 10H), 7.5-7.2 (m, 10H), 2.12 (s, 16H)
(5) Synthesis of Intermediate Product 11-5
   Dissolving Intermediate Product 11-4 (0.95 g, 1.37 mmol) into dimethylformamide (10 milliliter) and adding N-bromosuccinimide (0.29 g, 1.65 mmol), the resultant solution was stirred at room temperature for 4 hours. After adding water (100 milliliter) to the resultant reaction solution, and after filtering a precipitate from the solution, the precipitate was washed with the use of ethanol. The resultant solid was refined by means of a silica gel column chromatography (dissolution solvent: methylene chloride / hexane= 1/3), and as a result, Intermediate Product 11-5 was obtained. The result of the measurement in accordance with 90MHz ¹H-NMR is shown as the following:
   Produced Amount: 0.90 g, Yield: 85.3 %
   ¹H-NMR (CDCl₃): δ (ppm) 8.69 (d, 2H), 8.2-7.2 (m, 23H), 2.12 (s, 16H)
(6) Synthesis of 1-(10-(4-(2,2-diphenyl vinyl) phenyl) anthracene-9-yl)-3,5-di (spiro [cyclopentane-1,9'-fluorene-2'-yl]) benzene (Compound (A11))
   Intermediate Product 11-5 (0.81 g, 1.05 mmol), 4-(2,2-diphenyl vinyl) phenylboronic acid (0.38 g, 1.26 mmol), Pd(PPh₃)₄ (36 *µ*g, 32 *µ* mol), toluene (5 milliliter) and an aqueous solution (1.5 milliliter) of sodium carbonate (0.33 g, 3.15 mmol) are placed into a three neck flask already replaced with argon gas, and the resultant solution was heated at the temperature of 80 °C for 9 hours. Water (100 milliliter) and toluene (100 milliliter) were added to the resultant reaction solution, and an organic layer was separated, followed by drying with the use of anhydride magnesium sulfide. The dried organic layer was vacuum concentrated by means of an evaporator and the resultant solid was refined by means of a silica gel column chromatography (dissolution solvent: methylene chloride / hexane= 1/3), and as a result, Compound (A11) was obtained. It was confirmed in accordance with 90MHz ¹H-NMR and Field Desorption Mass Spectrometry (FD-MS) that the obtained crystals were the aimed compound. The result of the measurement in accordance with FD-MS is shown as the following:
   Produced Amount: 0.31 g, Yield: 31 %
   ¹H-NMR (CDCl₃): δ (ppm) 8.2-7.2 (m, 40H), 2.12 (s, 16H)
   FD-MS: calcd for C₇₄H₅₆ = 944, found m/z = 944 (M+, 100).

### Example 2 (Synthesis of Compound (A12))

The route for synthesis of Compound (A12) is shown as the following. 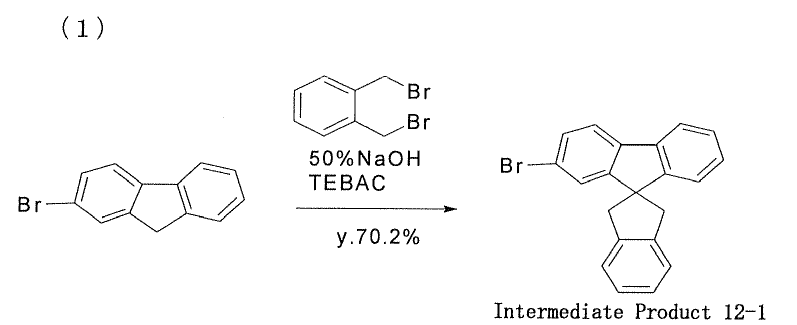 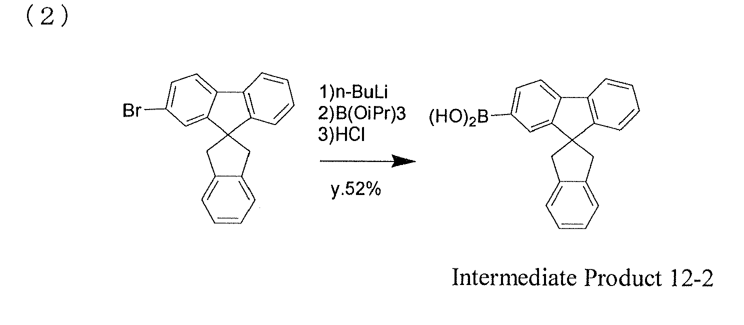 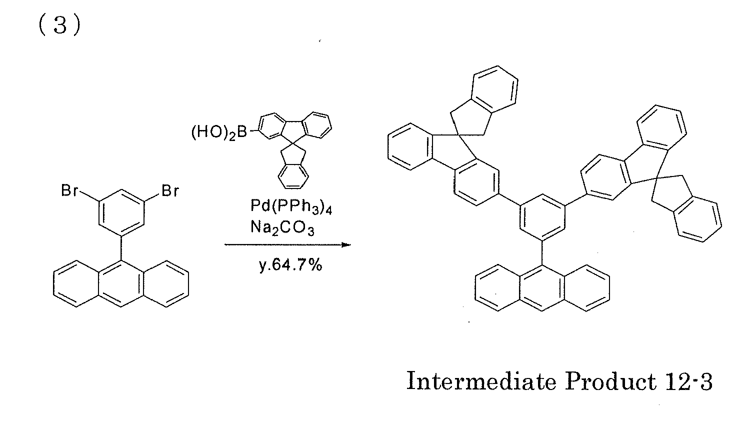 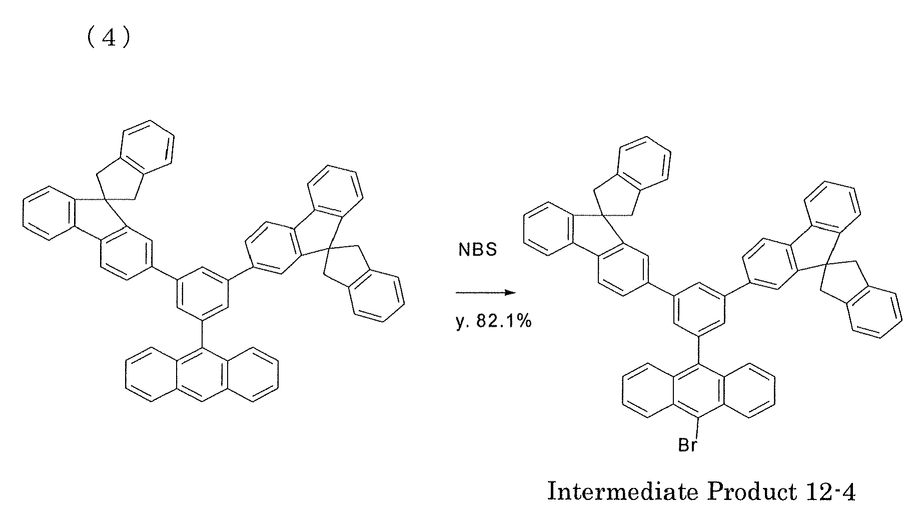 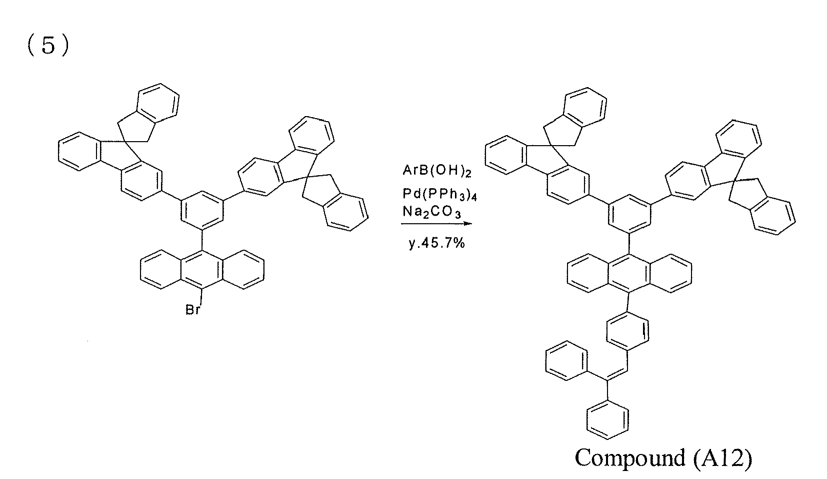
(1) Synthesis of Intermediate Product 12-1
   Into a three neck flask with a capacity of 100 milliliter and already replaced with argon gas, 2-bromo fluorene (10 g, 40. 8mmol), a dimethylsulfoxide (15 milliliter), benzyltriethylammonium chloride (0.19 g, 0.82 mmol) and α, α'-dibromo xylene (10.8 g, 40.8 mmol) were added and further, an aqueous solution (6.5 milliliter) of sodium hydroxide (50 % by weight) was dripped while stirring into the resultant solution, followed by further stirring at the temperature of 80 °C for 2 days. Further, water (100 milliliter) and toluene (100 milliliter) were added to the resultant reaction solution and an organic layer was separated. The organic layer was dried with the use of anhydride magnesium sulfide, vacuum concentrated by means of an evaporator and as a result, Intermediate Product 12-1 was obtained.
   Produced Amount: 9.95 g, Yield: 70.2 %
(2) Synthesis of Intermediate Product 12-2
   Synthesis was carried out similarly as Example 1, (3) except that Intermediate Product 12-1 (5.78 g, 16.7 mmol) was used instead of Intermediate Product 11-2, and Intermediate Product 12-2 was obtained.
   Produced Amount: 2.71 g, Yield: 52 %
(3) Synthesis of Intermediate Product 12-3
   Synthesis was carried out similarly as Example 1, (4) except that Intermediate Product 12-2 (1.77 g, 5.68 mmol) was used instead of Intermediate Product 11-3, and Intermediate Product 12-3 was obtained.
   Produced Amount: 1.47 g, Yield: 64.7 %
(4) Synthesis of Intermediate Product 12-4
   Synthesis was carried out similarly as Example 1, (5) except that Intermediate Product 12-3 (1.08 g, 1.37 mmol) was used instead of Intermediate Product 11-4, and Intermediate Product 12-4 was obtained.
   Produced Amount: 0.97 g, Yield: 82.1 %
(5) Synthesis of 1-(10-(4-(2,2-diphenyl vinyl) phenyl) anthracene-9-yl)-3,5-di (spiro [indane-2, 9 'fluorene-2'-yl]) benzene (Compound (A12))

Synthesis was carried out similarly as Example 1, (6) except that Intermediate Product 12-4 (0.91 g, 1.05 mmol) was used instead of Intermediate Product 11-5, and Compound (A12) was obtained. It was confirmed in accordance with FD-MS that Compound (A12) was the aimed compound. The result of the measurement in accordance with FD-MS is shown as the following:
Produced Amount: 0.50 g, Yield: 45.7 %
FD-MS: calcd for C₈₂H₉₆= 1040, found m/z = 1040 (M+, 100).

### Example 3 (Synthesis of Compound (A26))

The route for synthesis of Compound (A26) is shown as the following.

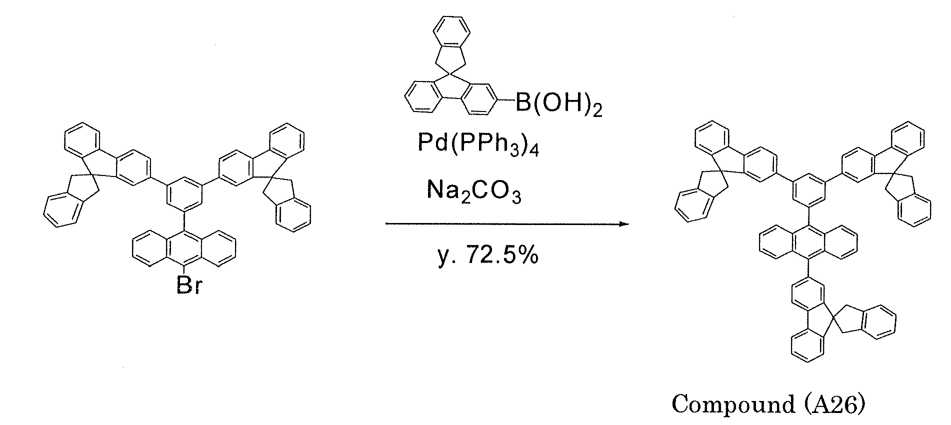

### (1) Synthesis of 1-(10-(spiro [indane-2,9'-fluorene-2'-yl])) anthracene-9-yl)-3,5-di(spiro [indane-2,9'-fluorene-2'-yl]) benzene (Compound A26)

Synthesis was carried out similarly as Example 2, (5) except that Intermediate Product 12-2 (0.39 g, 1.26 mmol) was used instead of 4-(2,2-diphenyl vinyl) phenylboronic acid, and Compound (A26) was obtained. It was confirmed in accordance with FD-MS that Compound (A26) was the aimed compound. The result of the measurement in accordance with FD-MS is shown as the following:
Produced Amount: 0.80 g, Yield: 72.5 %
FD-MS: calcd for C₈₃H₅₆= 1052, found m/z = 1052 (M+ , 100).

### Example 4 (Synthesis of Compound (A61))

The route for synthesis of Compound (A61) is shown as the following. 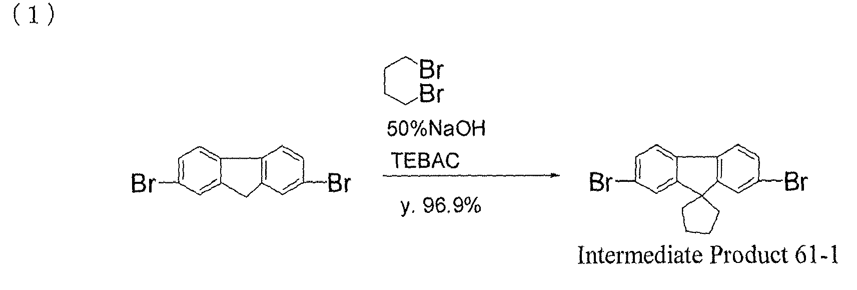 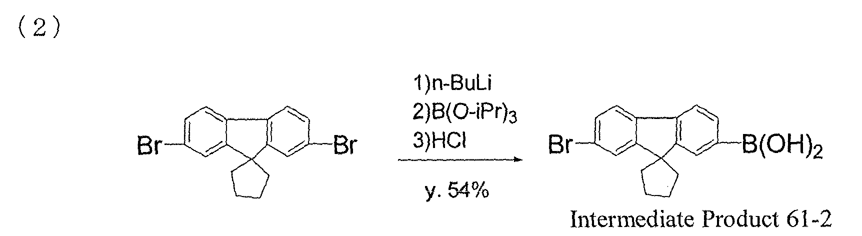 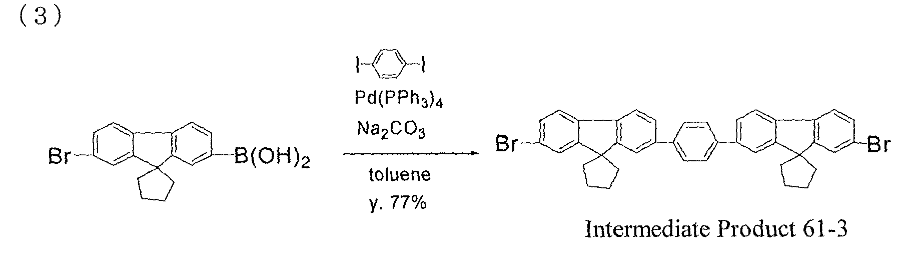 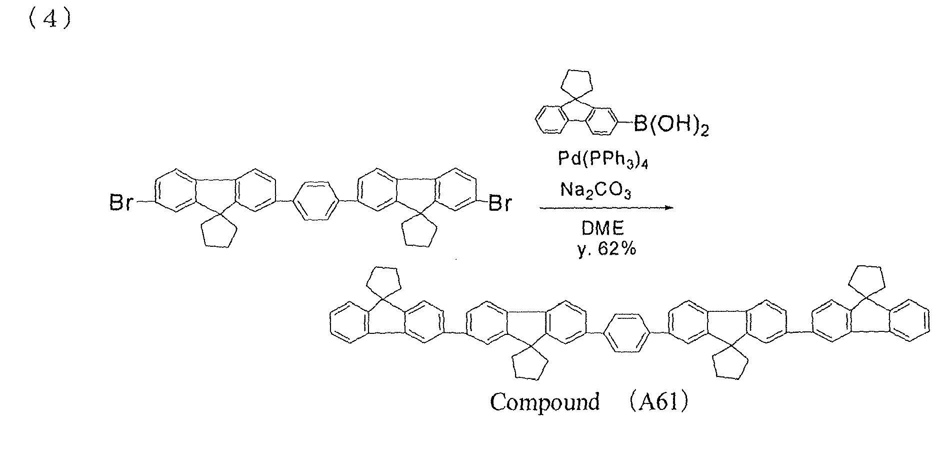
(1) Synthesis of Intermediate Product 61-1
   Into a three neck flask with a capacity of 100 milliliter and already replaced with argon gas, 2-bromo fluorene (2.0 g, 6.17 mmol), dimethylsulfoxide (3 milliliter) and benzyltriethylammonium chloride (0.031 g, 0.136 mmol) were placed and further, an aqueous solution (1 milliliter) of sodium hydroxide (50 % by weight) was dripped while stirring into the resultant solution. Subsequently, 1,4-dibromobutane (1.33 g, 6.17 mmol) was added and the resultant solution was stirred for 5 hours. Further, water (100 milliliter) and toluene (100 milliliter) were added to the resultant reaction solution and an organic layer was separated. The organic layer was dried with the use of anhydride magnesium sulfide, vacuum concentrated by means of an evaporator and as a result, a solid was obtained. By washing the resultant solid with the use of methanol, Intermediate Product 61-1 was obtained. Measured value of 90MHz ¹H-NMR is shown as the following:
   Produced Amount: 2.26 g, Yield: 96.9 %
   ¹H-NMR (CDCl₃)" δ (ppm) 7.6-7.4 (m, 6H), 2.09 (s, 8H)
(2) Synthesis of Intermediate Product 61-2
   Intermediate Product 61-1 (5.0 g, 13.2 mmol), anhydrous toluene (10 milliliter) and anhydrous ether (40 milliliter) were placed into a flask with a capacity of 300 milliliter and already replaced with argon gas, and further, after cooling the resultant solution down to -60 °C, a hexane solution (8.3 milliliter, 13.2 mmol) of n-butyllithium 1.59 M was poured into the cooled solution. The resultant reaction solution was stirred at the temperature of -20 °C for 1 hour and further, the solution was cooled down to ―60 °C, and then, adding anhydrous ether solution (40 milliliter) of triisopropyl boride (4.97 g, 26.4 mmol), the resultant solution was stirred for 1 hour. The temperature of the reaction solution was elevated little by little up to the room temperature, and the reaction solution was stood alone for a night. After adding 2N hydrochloric acid (100 milliliter) to the reacted solution, and after stirring the solution at room temperature for 1 hour, an organic layer was separated. The organic layer was dried with the use of anhydride magnesium sulfide, vacuum concentrated by means of an evaporator. The solid obtained after the extraction and the vacuum concentration was refined with silica gel column chromatography (dissolution solvent: methylene chloride /hexane= 2/1) and as a result, Intermediate Product 61-2 was obtained.
   Produced Amount: 2.44 g, Yield: 54 %
(3) Synthesis of Intermediate Product 61-3
   Intermediate Product 61-2 (2.0 g, 5.83 mmol), 1,4-diiodo benzene (0.77 g, 2.33 mmol, Pd(PPh₃)₄ (0.16 g, 0.14 mmol), toluene (15 milliliter) and an aqueous solution (7.5 milliliter) of sodium carbonate (1.48 g, 14.0 mmol) were placed into a three neck flask already replaced with argon gas, and the resultant solution was heated at the temperature of 80 °C for 9 hours. Water (100 milliliter) and toluene (100 milliliter) were added to the resultant reaction solution, and an organic layer was separated, followed by drying with the use of anhydride magnesium sulfide. The resultant solid was refined by means of a silica gel column chromatography (dissolution solvent: methylene chloride / hexane= 1/3), and as a result, Intermediate Product 61-3 was obtained.
   Produced Amount: 1.21 g, Yield: 77 %
(4) Synthesis of 1,4-di (spiro [cyclopentane-1,9'-fluorene-7'-(spiro [cyclopentane-1,9'-fluorene-2'-yl) 2'-yl]) benzene (Compound (A61)

Intermediate Product 61-3 (1.0 g, 1.5 mmol), Intermediate Product 11-3 (1.0 g, 3.75 mmol), Pd(PPh₃)₄ (0.17 g, 0.15 mmol), dimethoxyethane (DME) (1 milliliter) and an aqueous solution (4.5 milliliter) of sodium carbonate (0.95 g, 8.96 mmol) were placed into a three neck flask already replaced with argon gas, and the resultant solution was heated at the temperature of 80 °C for 9 hours. Water (100 milliliter) and toluene (100 milliliter) were added to the resultant reaction solution, and an organic layer was separated, followed by drying with the use of anhydride magnesium sulfide. The dried organic layer was vacuum concentrated by means of an evaporator and the resultant solid was refined by means of a silica gel column chromatography (dissolution solvent: methylene chloride / hexane= 1/3), and as a result, Compound (A61) was obtained. It was confirmed in accordance with FD-MS that Compound (A61) was the aimed compound. The result of the measurement in accordance with FD-MS is shown as the following:
Produced Amount: 0.88 g, Yield: 62 %
FD-MS: calcd for C₇₄H₆₂= 950, found m/z = 950 (M+ , 100).

### Example 5 (Synthesis of Compound (A31))

The route for synthesis of Compound (A31) is shown as the following. 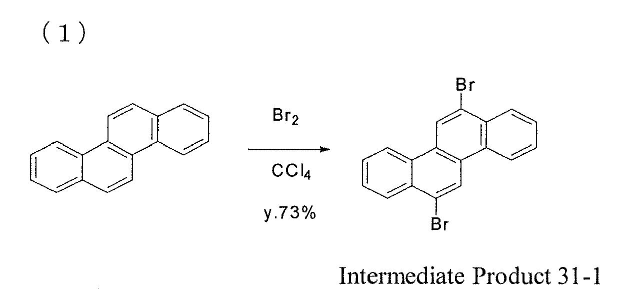 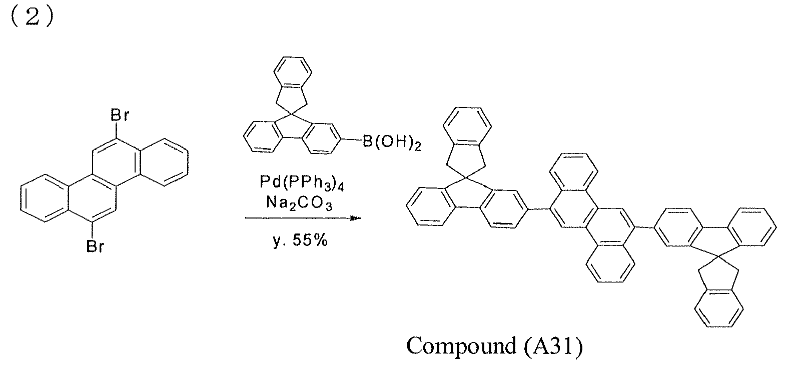
(1) Synthesis of Intermediate Product 31-1
   Tetrachloromethane (400 milliliter) and chrysene (15 g, 66 mmol) were placed into a flask and then, bromine / tetrachloromethane (10 milliliter, 0.19 mol /100 milliliter) was dripped slowly at the room temperature. While agitating the resultant solution intensely, it was refluxed for 5 hours. After the reacted solution was cooled, precipitated solids were separated by filtration and washed with methanol. Re-crystallization was carried out with the use of toluene, and as a result, Intermediate Product 31-1 was obtained. Measured value of 90MHz ¹H-NMR is shown as the following:
   Produced Amount: 18.5 g, Yield: 73 %
   ¹H-NMR (CDCl₃): δ (ppm) 9.0(s, 2H), 8.8-8.7(m, 2H), 8.5-8.4(m, 2H), 7.9-7.7 (m, 4H)
(2) Synthesis of 6,12-di (spiro [indane-2, 9'-fluorene-2'-yl]) chrysene (Compound (A31))
   Synthesis was carried out similarly as Example 4 (4) except that Intermediate Product 31-1 (0.58 g, 1.5 mmol) was used instead of Intermediate Product 61-3, and that Intermediate Product 12-2 (1.17 g, 3.75 mmol) was used instead of Intermediate Product 11-3, and as a result, Compound (A31) was obtained. It was confirmed in accordance with FD-MS that Compound (A31) was the aimed compound. The result of the measurement in accordance with FD-MS is shown as the following:
   Produced Amount: 0.63 g, Yield: 55 %
   FD-MS: calcd for C₆₀H₄₀= 760, found m/z = 760 (M+ , 100).

### Example 6 (Synthesis of Compound (A35))

The route for synthesis of Compound (A35) is shown as the following. 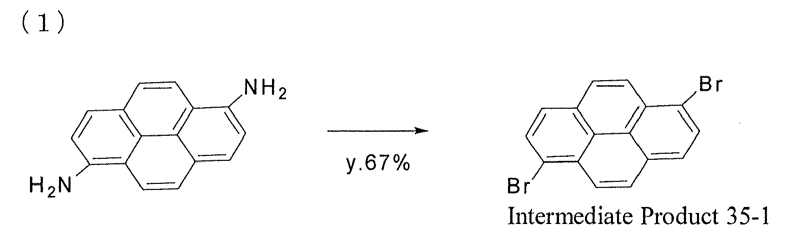 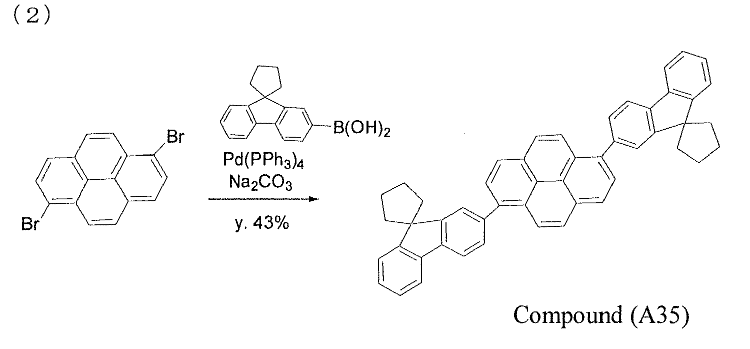
(1) Synthesis of Intermediate Product 35-1
   Into a beaker, 1,6-diamino pyrene (8.75 g, 37.5 mmol) was placed, adding 47 % HBr (60 milliliter) and after cooling it down to 0 °C, an aqueous solution (10 milliliter) of NaNO₂ (2.70 g, 37.5 mmol) was further added and the resultant solution was stirred. The resultant solution was added into a flask containing CuBr (2.96 g) and 47 % HBr (3 milliliter), and the resultant solution was stirred at the temperature of 60 °C for 30 minutes. Extracting from the reacted solution with the use of toluene, a crude product was obtained by means of vacuum concentration. The resultant crude product was refined by means of a silica gel column chromatography (dissolution solvent: hexane / methylene chloride= 3/1), and as a result, Intermediate Product 35-1 was obtained.
   Produced Amount: 7.43 g, Yield: 55 %
(2) Synthesis of 1,6-di (spiro [cyclopentane-1,9'-fluorene-2'-yl]) pyrene (Compound (A35))
   Synthesis was carried out similarly as Example 4, (4) except that Intermediate Product 35-1 (0.58 g, 1.5 mmol) was used instead of Intermediate Product 61-3, and Compound (A35) was obtained. It was confirmed in accordance with FD-MS that Compound (A35) was the aimed compound. The measurement results are shown as the following:
   Produced Amount: 0.41 g, Yield: 43 %
   FD-MS: calcd for C₅₀H₃₈= 638, found m/z = 638 (M+, 100).

### Example 7 (Fabrication of an organic EL device)

A glass substrate (manufactured by GEOMATEC Company) of 25 mmx75 mmx1.1 mm thickness having an ITO transparent electrode was cleaned by application of ultrasonic wave in isopropyl alcohol for 5 minutes and then by exposure to ozone generated by ultraviolet light for 30 minutes. On the substrate, a film of polyethylene dihydroxy thiophene (PEDOT) for the use of the hole injecting layer with film thickness of 100 nm was formed in accordance with a spin coat process and then, a solution prepared by dissolving Compound (A11) synthesized as a material for luminous coated film was applied over PEDOT by means of a spin coat process to form a light emitting layer. The film thickness was 50 nm. On the film formed above, a film of tris(8-quinolinol)aluminum (Alq film) having a thickness of 10 nm was formed. The formed film of Alq worked as the electron transporting layer. Thereafter, Li (the source of lithium: manufactured by SAES GETTERS Company) as a reductive dopant and Alq were binary vapor deposited and an Alq:Li film was formed as the electron injecting layer (or the cathode). On the Alq:Li film, metallic aluminum was vapor deposited to form a metal cathode and an organic El device was prepared.

Luminance and Current Efficiency of the fabricated device were measured and further, a sate of light emission about the light emitting surface after storing the device at an elevated temperature (after storing at the temperature of 120 °C for 50 hours) was observed. The results are shown in Table 1.

### Examples 8 to 10

Organic EL devices were fabricated similarly as Example 7 except that compounds described in Table 1 were used instead of Compound (A11). Luminance and Current Efficiency of the fabricated devices were measured and further, sates of light emission about the light emitting surface after storing the device at an elevated temperature (after storing at the temperature of 120 °C for 50 hours) were observed. The results are shown in Table 1.

### Comparative Example 1

An organic EL devices was fabricated similarly as Example 7 except that Compound (H1) below was used instead of Compound (A11). Luminance and Current Efficiency of the fabricated device were measured and further, a sate of light emission about the light emitting surface after storing the device at an elevated temperature (after storing at the temperature of 120 °C for 50 hours) was observed. The results are shown in Table 1.

### Comparative Example 2

An organic EL device was fabricated similarly as Example 7 except that Compound (H2) below was used instead of Compound (A11). Luminance and Current Efficiency of the fabricated device were measured and further, a sate of light emission about the light emitting surface after storing the device at an elevated temperature (after storing at the temperature of 120 °C for 50 hours) was observed. The results are shown in Table 1. Additionally, C₅ in Compound (H2) represents a n-pentyl group.

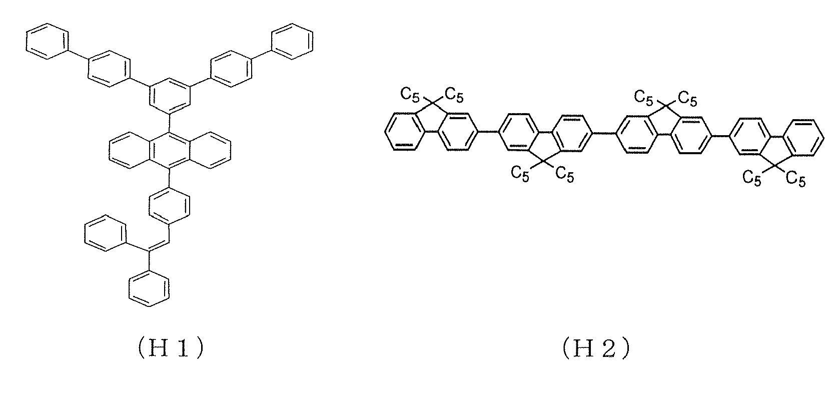

### Example 11

A glass substrate (manufactured by GEOMATEC Company) of 25 mm×75 mm×1.1 mm thickness having an ITO transparent electrode was cleaned by application of ultrasonic wave in isopropyl alcohol for 5 minutes and then by exposure to ozone generated by ultraviolet light for 30 minutes. The glass substrate having the transparent electrode lines which had been cleaned was attached to a substrate holder of a vacuum vapor deposition apparatus. On the surface of the cleaned substrate at the side having the transparent electrode, a film of N,N'-bis (N,N'-diphenyl-4-aminophenyl)-N,N'-diephenyl-4,4'-diamino-1,1'-biphenyl (film of TPD232) having a thickness of 60 nm was formed so that the formed film covered the transparent electrode. The formed film of TPD232 worked as the hole injecting layer. Successively, a film of N,N',N'-tetra(4-biphenyl)-diamino biphenylene (TBDB film) with a film thickness of 20 nm was formed over the film of TPD232. The formed film worked as the hole transporting layer. Furthermore, Compound (A31) as a host material was vapor deposited to form a film with a thickness of 40 nm. At the same time, a dopant of the following arylamine compound was vapor-deposited as a light emitting molecule in weight ratio of the dopant : Compound (A31) = 3:40. The formed film worked as a light emitting layer. On the film formed above, a film of Alq having a thickness of 10 nm was formed. The formed film worked as an electron transporting layer. Thereafter, Li (the source of lithium: manufactured by SAES GETTERS Company) as a reductive dopant and Alq were binary vapor deposited and an Alq:Li film (film thickness: 10 nm) was formed as the electron injecting layer (or the cathode). On the Alq:Li film, metallic aluminum was vapor deposited to form a metal cathode and an organic El device was prepared.

### Dopant:

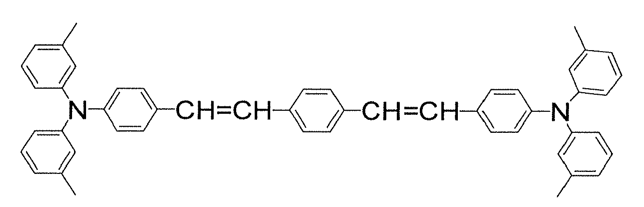

Current Efficiency of the fabricated device was measured and further, a sate of light emission about the light emitting surface after storing the device at an elevated temperature (after storing at the temperature of 120 °C for 50 hours) was observed. The results are shown in Table 2.

### Example 12

Organic EL devices were fabricated similarly as Example 11 except that compounds described in Table 1 were used instead of Compound (A31). Luminance and Current Efficiency of the fabricated devices were measured and further, sates of light emission about the light emitting surface after storing the device at an elevated temperature (after storing at the temperature of 120 °C for 50 hours) were observed. The results are shown in Table 2.

### Comparative Example 3

An organic EL device was fabricated similarly as Example 11 except that Compound (H3) below was used instead of Compound (A31). Luminance and Current Efficiency of the fabricated device were measured and further, a sate of light emission about the light emitting surface after storing the device at an elevated temperature (after storing at the temperature of 120 °C for 50 hours) was observed. The results are shown in Table 2.

### Comparative Example 4

An organic EL device was fabricated similarly as Example 11 except that Compound (H4) below was used instead of Compound (A31). Luminance and Current Efficiency of the fabricated device were measured and further, a sate of light emission about the light emitting surface after storing the device at an elevated temperature (after storing at the temperature of 120 °C for 50 hours) was observed. The results are shown in Table 2.

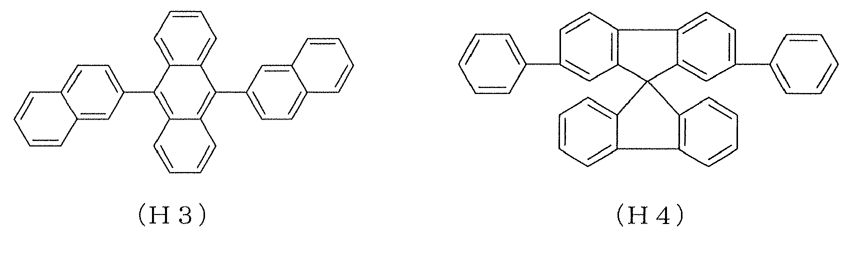

**Table 1**

| | Organic Material in Light emtting layer | Applied Voltage (V) | Luminance (cd/m2) | Current Efficiency (Lumen/W) | Surface state of light emitting face after storing at the temperature of 120 °C for 50 hours |
|---|---|---|---|---|---|
| Ex. 7 | A11 | 5.2 | 130 | 1.6 | Emitting uniform blue light |
| Ex. 8 | A12 | 5.4 | 150 | 1.8 | Emitting uniform blue light |
| Ex. 9 | A26 | 5.2 | 160 | 1.9 | Emitting uniform blue light |
| Ex. 10 | A61 | 5.2 | 130 | 1.5 | Emitting uniform blue light |
| Co. Ex. 1 | H1 | 7.1 | 100 | 0.9 | Crystal growth existed |
| Co. Ex. 2 | H2 | 5.5 | 120 | 1.2 | Color change existed |

**Table 2**

| | Organic host Material in Light emtting layer | Applied Voltage (V) | Current Efficiency (Lumen/W) | Surface state of light emitting face aftet storing at the temperature of 120 °C for 50 hours |
|---|---|---|---|---|
| Ex. 11 | A31 | 5.3 | 4.6 | Emitting uniform blue light |
| Ex. 12 | A35 | 5.6 | 5.2 | Emitting uniform blue light |
| Co. Ex. 3 | H3 | 7.4 | 2.1 | Crystal growth existed |
| Co. Ex. 4 | H4 | 7.3 | 3.1 | Emitting uniform blue light |

Exactly as Examples 7 to 12 indicate in Tables 1 and 2, they verify that the organic EL devices which employ a compound having a spiro bond of the present invention exhibit an excellent luminance, current efficiency and pure blue light emission even under a low driving voltage, and also reveals a uniform blue light emission because they have heat resistance against elevated temperatures. However, exactly as Comparative Examples 1 to 3 indicate in Tables 1 and 2, they verify that the organic EL devices which do not employ the compound having a spiro bond or which employ a compound with a fluorenyl group which does not have a spiro bond exhibit poor luminance and current efficiency because crystallizations in the thin film occurs with high-temperature, or the wave length of the light emission shifts to longer than blue light because molecules tend to aggregate each other.

Although the present invention seeks to provide a novel compound for emitting blue light stably and not conventional, it is important for the invention to introduce a group having an adequate spiro bond into an adequate site. Particularly, considerations are necessary for introducing a spirobifluorenyl group because, for example, in a case where X is a group having chrysenylene backbone structure, pyrenylene backbone structure or phenanthlene backbone structure and Sp and Y are spirobifluorenyl groups in the general formula (1), a vapor deposition temperature in device fabrication becomes high causing infusion of thermally decomposed substances into a thin film, resultantly inducing a shift in the color of the light emission. Further, exactly as Comparative Example 4 indicates, in a case where X is a spirobifluorenylene group, although the crystallization of the thin film is suppressed, distances between the molecules become so remote that mobility of charges fall and as a result, the organic EL device requires high driving voltage to work favorably.

### INDUSTRIAL APPLICABILITY

The organic EL device employing either the compound having a spiro bond or the material for forming luminous coated film in accordance with the present invention is superior in heat resistance, has stability of the thin film composing the device, emits uniform blue light, and exhibits an excellent luminance, current efficiency even under a low driving voltage. Therefore, they are highly applicable as the organic EL devices having practical performance.

## Claims

1. A compound having a spiro bond represented by a following general formula (1):
(Sp-)ₙ X(-Y)ₘ (1)
wherein Sp is a group having a spiro bond represented by a following general formula (2): 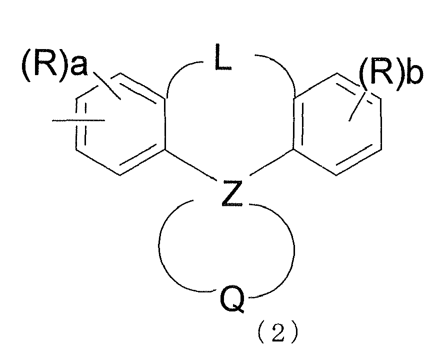
wherein L represents a single bond, ― (CR'R") ₑ ―,― (Si R' R")ₑ ―, -O-, -CO- or -NR'-;
R' and R" each independently represents a hydrogen atom, a substituted or unsubstituted aromatic group having 6 to 50 ring carbon atoms, a substituted or unsubstituted heterocyclic group having 5 to 50 ring atoms, or a substituted or unsubstituted alkyl group having 1 to 50 carbon atoms; e represents an integer of 1 to 10; further R' and R" may be the same with or different from each other;
Z represents a carbon atom, a silicon atom or a germanium atom;
Q represents a group forming a ring structure;
R represents a substituted or unsubstituted aromatic group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, a carboxyl group, a halogen atom, a cyano group, a nitro group or a hydroxyl group; when there are plural of R, they may be the same with or different from each other and they may be bond with each other to form a ring structure; a and b each independently represents an integer of 0 to 4;
X represents a substituted or unsubstituted aromatic group having 6 to 50 ring carbon atoms, a substituted or unsubstituted condensed aromatic ring group having 12 to 20 ring carbon atoms, a substituted or unsubstituted aromatic heterocyclic group having 5 to 50 ring atoms or a group formed by combining plural of the preceding groups; excluding a case where X is an anthracendiyl group or a polyanthracendiyl group;
Y represents a substituted or unsubstituted aromatic group having 6 to 50 ring carbon atoms and may further having a vinyl-bond and still further may contain a group having a spiro bond represented by the general formula (2);
n represents an integer of 1 to 4;
m represents an integer of 1 to 2; and
when Sp in the general formula (1) is a spirobifluorenyl group, a case where X has a backbone structure selected from a group consisting of pyrenylene backbone structure, chrysenylene backbone structure and phenanthlene backbone structure is excluded.

2. The compound having a spiro bond according to Claim 1, wherein Sp in the general formula (1) is represented by the following general formula (3): 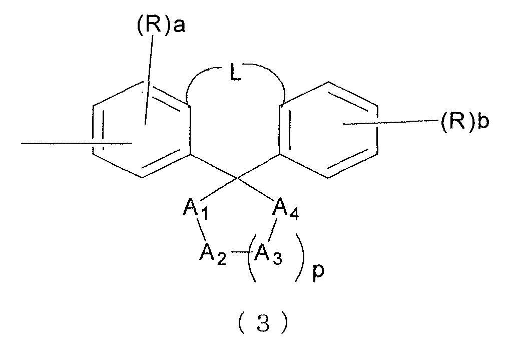
wherein R represents a substituted or unsubstituted aromatic group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, a carboxyl group, a halogen atom, a cyano group, a nitro group or a hydroxyl group;
L represents a single bond, - (CR'R") ₑ ―, ― (SiR'R") ₑ ―, ―O―,
―CO― or ―NR'―;
a and b each independently represents an integer of 0 to 4;
A₁ to A₄ each independently represents ―CR'R"―, ―SiR'R"―, ―O―, ―NR'― or ―CO―;
R' and R" each independently represents a hydrogen atom, a substituted or unsubstituted aromatic group having 6 to 50 ring carbon atoms, a substituted or unsubstituted heterocyclic group having 5 to 50 ring atoms, or a substituted or unsubstituted alkyl group having 1 to 50 carbon atoms; R' and R" may be the same with or different from each other and they may bond with each other to form a ring structure; and
p represents an integer of 1 to 10.

3. The compound having a spiro bond according to Claim 2, wherein at least two adjacent components among A₁ to A₄ in the general formula (3) each represents ―CR'R"―;
R' and R" each independently represents a hydrogen atom, a substituted or unsubstituted aromatic group having 6 to 50 ring carbon atoms, a substituted or unsubstituted heterocyclic group having 5 to 50 ring atoms, or a substituted or unsubstituted alkyl group having 1 to 50 carbon atoms; R' and R" may be the same with or different from each other and they may bond with each other to form a ring structure; and
the adjacent R's, the adjacent R"s or both R' and R" will bond saturatedly or unsaturatedly forming a ring structure having 4 to 50 carbon atoms as a result.

4. The compound having a spiro bond according to Claim 1, wherein Sp is a group represented by any one of the following general formulae (4) to (7): 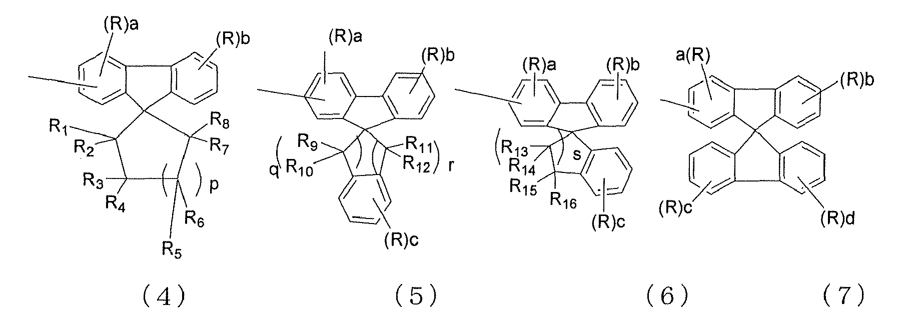
wherein R represents a substituted or unsubstituted aromatic group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 7 to 50 ring atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, a carboxyl group, a halogen atom, a cyano group, a nitro group or a hydroxyl group; when there are plural of R, they may be the same with or different from each other and they may be bond with each other to form a ring structure; and
R₁ to R₁₆ each independently represents a hydrogen atom, a substituted or unsubstituted aromatic group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, a carboxyl group, a halogen atom, a cyano group, a nitro group or a hydroxyl group; at least two among R₁ to R₁₆ may bond each other to form a ring structure;
a, b, c and d each represents an integer of 0 to 4 respectively;
p, q, r and s each represents an integer number of 1 to 10 respectively;
wherein X is a group represented by any one of the following general formula e (8) to (25) or a group made by combining at least two of groups represente d by the following general formulae (8) to (25): 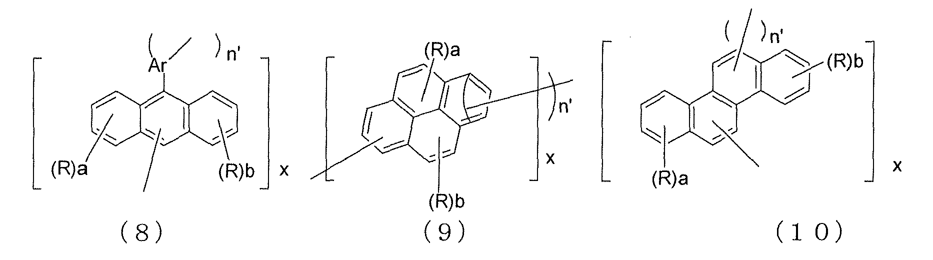 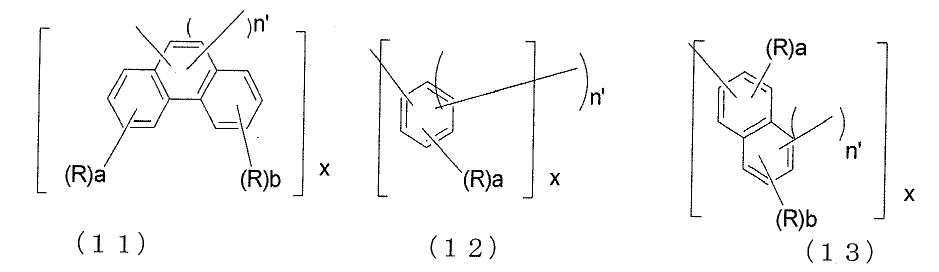 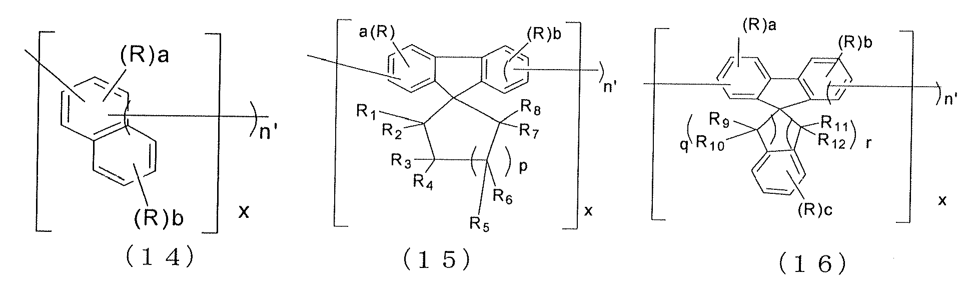 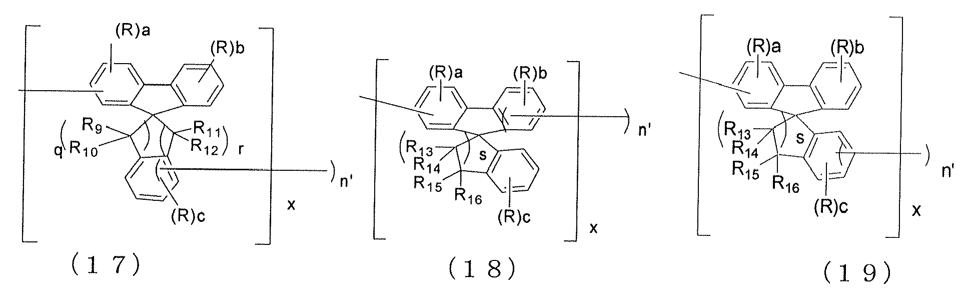 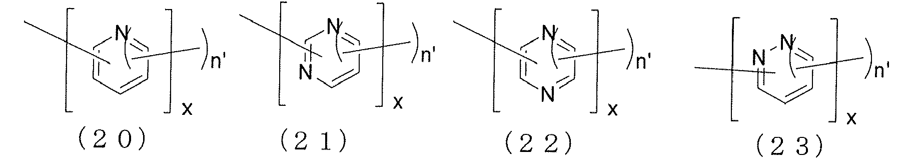 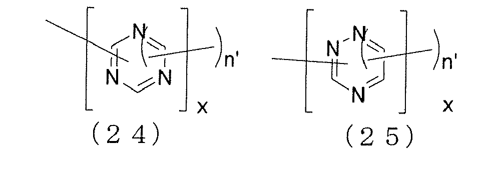
wherein R, R₁ to R₁₆, a to d and p to s are the same as the foregoing description;
wherein Ar represents a substituted or unsubstituted aromatic group having 6 to 50 ring carbon atoms, a substituted or unsubstituted aromatic heterocyclic group having 5 to 50 ring atoms, or a group made by combining plural of those preceding groups; excluding a case where Ar is an anthracendiyl group or a polyanthracendiyl group;
n' represents an integer of 0 to 5;
x represents an integer of 1 to 20; and
when Sp is a group represented by the general formula (7), a case where X is a group represented by any one of the general formulae (9) to (11) is excluded.

5. The organic electroluminescence device according to Claim 4, wherein Y in the general formula (1) is a group represented by a general formula (26): 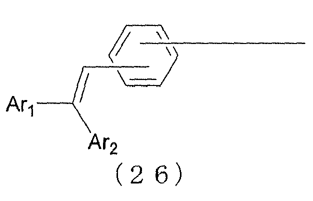
wherein Ar₁ and Ar₂ each independently represents a substituted or unsubstituted aromatic group having 6 to 50 ring carbon atoms respectively and further, Ar₁ and Ar₂ may be the same with or different from each other.

6. A compound having a spiro bond according to any one of Claims 1 to 5, which is a light emitting material for an organic electroluminescence device.

7. A material for forming a luminous coated film which comprises the compound having a spiro bond according to any one of Claims 1 to 5.

8. An organic electroluminescence device which comprises at least one organic thin film layer sandwiched between a pair of electrode consisting of an anode and a cathode, wherein the organic thin film layer comprises the compound having a spiro bond according to any one of Claims 1 to 5.

9. The organic electroluminescence device according to Claim 8, wherein said light emitting layer comprises the compound having a spiro bond.

10. The organic electroluminescence device according to Claim 8, which emits bluish light.

11. The organic electroluminescence device according to Claim 9, which emits bluish light.
